# EUROPEAN PATENT APPLICATION

(11) **EP 4 130 283 A1**
(43) Date of publication of application: **08.02.2023**
(21) Application number: 21752873.6
(22) Date of filing: 14.02.2021
(51) Int. Cl.: C12N 15/90, A61K 31/7088, A61K 48/00, A61P 35/00, C12N 15/31, C12N 15/869, C12Q 1/6886, C12Q 1/6897, G01N 33/15, G01N 33/50, G01N 33/68

(54) **NUCLEIC ACID CONSTRUCT THAT CAN MEASURE HOMOLOGOUS RECOMBINATION ACTIVITY, AND METHOD FOR USING SAME**

(30) Priority: 14.02.2020 JP 2020023875
(71) Applicant: Public University Corporation Yokohama City University, Yokohama-shi, Kanagawa 236-0027 (JP)
(72) Inventor: ADACHI, Noritaka, Yokohama-shi, Kanagawa 236-0027 (JP)
(74) Representative: Mewburn Ellis LLP
(86) International application number: PCT/JP2021/005381
(87) International publication number: WO 2021/162120

(57) **Abstract**

Disclosed are a means which enables simple and rapid detection of the presence or absence, and of the degree, of homologous recombination activity in an individual, and a means which is useful for detection and treatment of homologous recombination-restored cancer for which no treatment means is available at present. The nucleic acid construct of the present invention comprises: (1) a promoter region; (2) a mutant gene sequence containing a cleavage site in a gene sequence encoding a protein; and (3) a base sequence composed of a first homologous region and a second homologous region, the base sequence being capable of replacing, by homologous recombination, a partial region in the mutant gene sequence of (2), the partial region containing the cleavage site. The nucleic acid construct of the present invention can be used as a measurement reagent or kit for homologous recombination activity, a diagnostic agent for homologous recombination-deficient cancer, a therapeutic agent for homologous recombination-restored cancer, or the like.

## Description

### TECHNICAL FIELD

The present invention relates to a nucleic acid construct capable of measuring homologous recombination activity, and use of the nucleic acid construct as a measurement reagent and measurement kit for homologous recombination activity, a detection agent for a homologous recombination-deficient cancer and the like.

### BACKGROUND ART

Double-strand breaks of genomic DNA are dangerous DNA lesions with the highest mutagenicity. Therefore, homologous recombination (HR), which is capable of accurately repairing double-strand breaks, are indispensable for maintaining stability of the genome (Non-Patent Document 1). HR occurs only in the S to G2 phases, during which sister chromatids as templates are present, whereas, non-homologous end joining (NHEJ) functions as a double-strand break repair mechanism besides HR throughout the cell cycle (Non-Patent Document 2). In addition to these, there are minor repair routes such as alternative end-joining, in which PolQ is involved, and single-strand annealing, but, in any of these routes, induction of mutations inevitably occurs after the repair (Non-Patent Document 3).

HR is a highly complex reaction involving a number of proteins. In particular, Rad51, which is involved in strand exchange reaction, plays a central role (Non-Patent Document 4). Further, factors that assist the function of Rad51, such as BRCA1 and BRCA2, are indispensable for HR, and human cells deficient in BRCA1 or BRCA2 exhibit abnormality in the HR repair of double-strand breaks (Non-Patent Document 5).

HR deficiency causes various cancers. Hereditary breast ovarian cancer (HBOC) is the hereditary tumor that is most frequently found. Its main cause is a genetic abnormality of BRCA1 or BRCA2 (Non-Patent Documents 6 and 7). Also for sporadic (non-genetic) cancers, mutations in HR-related genes have been reported in various types of cancers (Non-Patent Documents 8 and 9).

Diagnosis of HR-deficient cancers is carried out by genetic diagnosis on BRCA1 and BRCA2 (Non-Patent Document 10). However, from the viewpoint of the fact that several ten kinds of proteins are involved in the HR reaction, the current genetic diagnosis cannot be considered to be a sufficient examination method. Based on the idea that detection of the HR activity itself is more desirable, several methods have been proposed therefor (Non-Patent Documents 11 and 12). However, all of these are methods for relative evaluation of the HR activity. It is conceivable that the most effective and reliable method is evaluation of the HR activities of the individual cells as absolute values rather than relative evaluation. However, such a method has not been developed.

For treatment of HR-deficient cancers, platinum formulations or poly(ADPribose) polymerase (PARP) inhibitors are used (Non-Patent Documents 13 and 14). In particular, the latter, PARP inhibitors, are attracting attention since they have a "synthetic lethal" relationship with HR. However, frequent occurrence of resistant cancers in PARP inhibitor treatment has been a serious problem, and there is no method for treatment of such resistant cancers at present (Non-Patent Documents 15 and 16).

### PRIOR ART DOCUMENT(S)

### NON-PATENT DOCUMENT(S)

Non-Patent Document 1: Moynahan ME and Jasin M. Nat Rev Mol Cell Biol. 2010 Mar;11(3):196-207.
Non-Patent Document 2: Chang HHY et al. Nat Rev Mol Cell Biol. 2017 Aug;18(8):495-506.
Non-Patent Document 3: Saito S et al. Nat Commun. 2017 Jul 11;8:16112
Non-Patent Document 4: Morrical SW. Cold Spring Harb Perspect Biol. 2015 Feb 2;7(2):a016444.
Non-Patent Document 5: Prakash R et al. Cold Spring Harb Perspect Biol. 2015 Apr 1;7(4):a016600.
Non-Patent Document 6: King MC et al. Science. 2003 Oct 24;302(5645):643-646.
Non-Patent Document 7: Cancer Genome Atlas Research Network. Nature. 2011 Jun 29;474(7353):609-615.
Non-Patent Document 8: Heeke AL et al. JCO Precis Oncol. 2018;2018.
Non-Patent Document 9: Lord CJ and Ashworth A. Nat Rev Cancer. 2016 Feb;16(2):110-120.
Non-Patent Document 10: Gourley C et al. J Clin Oncol. 2019 Sep 1;37(25):2257-2269.
Non-Patent Document 11: Telli ML et al. Clin Cancer Res. 2016 Aug 1;22(15):3764-3773.
Non-Patent Document 12: Ransburgh DJ et al. Cancer Res. 2010 Feb 1;70(3):988-995.
Non-Patent Document 13: Bryant HE et al. Nature. 2005 Apr 14;434(7035):913-917.
Non-Patent Document 14: Farmer H et al. Nature. 2005 Apr 14;434(7035):917-921.
Non-Patent Document 15: Lord CJ and Ashworth A. Nat Med. 2013 Nov;19(11):1381-1388.
Non-Patent Document 16: Noordermeer SM and van Attikum H. Trends Cell Biol. 2019 Oct;29(10):820-834.
Non-Patent Document 17: Norquist B et al. J Clin Oncol. 2011 Aug 1;29(22):3008-3015.
Non-Patent Document 18: Bouwman P and Jonkers J. Clin Cancer Res. 2014 Feb 1;20(3):540-547.
Non-Patent Document 19: Pierce AJ et al. Genes Dev. 1999 Oct 15;13(20):2633-2638.
Non-Patent Document 20: Liang F et al. Proc Natl Acad Sci USA. 1996 Aug 20;93(17):8929-8933.

### SUMMARY OF THE INVENTION

### PROBLEMS TO BE SOLVED BY THE INVENTION

If there is a method that enables simple and rapid detection of the presence or absence, or the degree, of HR activity in cells, tissues, or individuals, the method is expected to be extremely useful for diagnosis and treatment of HR-deficient cancers including HBOC irrespective of the types of the cancers. Further, since recovery (reverse mutation) of BRCA1 is a cause of acquisition of PARP inhibitor resistance in BRCA1-deficient cancers (Non-Patent Documents 17 and 18), it would be a great advantage in the treatment of resistant cancers if detection and killing of cancer cells showing the recovery of HR activity become possible. However, at present, there is no known means of simply and rapidly detecting the presence or absence, or the degree, of the HR activity in an individual, or means of selectively detecting and killing cancer cells showing recovery of the HR activity.

An object of the present invention is to provide a means that enables simple and rapid detection of the presence or absence, or the degree, of the HR activity in an individual. Another object of the present invention is to provide a means that is useful for detection and treatment of homologous recombination-restored cancers for which no treatment means is available at present.

### MEANS FOR SOLVING THE PROBLEMS

As a result of intensive study, the present inventors developed a nucleic acid construct having a structure designed such that a protein-encoding gene sequence is reconstructed in a cell having homologous recombination activity to cause expression of the protein while neither the reconstruction of the gene sequence nor the expression of the protein occurs in a cell whose homologous recombination activity is lost. The present inventors then discovered that the nucleic acid construct enables detection of the presence or absence of homologous recombination activity using a transient expression system in a very short time, and enables evaluation/measurement of homologous recombination activity as an absolute value rather than relative evaluation, and that, by preparing the nucleic acid construct of the present invention using a gene sequence encoding a protein having an action to decrease cell survival rate, such as a suicide gene, homologous recombination-restored cancer cells can be killed to allow production of a therapeutic effect, thereby completing the present invention that includes the following modes.
[1] A nucleic acid construct comprising the following (1) to (3):
   (1) a promoter region;
   (2) a mutant gene sequence containing a cleavage site in a gene sequence encoding a protein; and
   (3) a base sequence composed of a first homologous region and a second homologous region and capable of replacing, by homologous recombination, a partial region in the mutant gene sequence of (2), the partial region containing the cleavage site, the base sequence being any one of the following (i) to (iii):
      (i) a continuous partial sequence in the gene sequence encoding the protein, the continuous partial sequence containing upstream and downstream regions adjacent to the cleavage site in the mutant gene sequence of (2);
      (ii) a base sequence which is homologous to the partial sequence of (i) and encodes the same amino acid sequence as that encoded by said partial sequence; and
      (iii) a continuous partial sequence in a gene sequence encoding a protein whose sequence is 80% or more identical to that of the previously-mentioned protein and which has the same activity as the previously-mentioned protein, the continuous partial sequence being a base sequence homologous to the partial sequence of (i).
[2] The nucleic acid construct according to claim 1, wherein the (2) contains a stop codon upstream of the cleavage site.
[3] The nucleic acid construct according to [1], wherein the sequence of (3) is a continuous partial sequence in the gene sequence encoding the protein, the continuous partial sequence containing upstream and downstream regions adjacent to the cleavage site in the mutant gene sequence of (2).
[4] The nucleic acid construct according to [2], wherein the sequence of (3) is a continuous partial sequence in the gene sequence encoding the protein, the continuous partial sequence containing the stop codon, and upstream and downstream regions adjacent to the cleavage site, in the mutant gene sequence of (2).
[5] The nucleic acid construct according to any one of [1] to [4], wherein the sequences of (ii) and (iii) is 90% or more homologous to the partial sequence of (i).
[6] The nucleic acid construct according to any one of [1] to [5], wherein each of the first homologous region and the second homologous region in the sequence of (3) has a strand length of at least 20 bases.
[7] The nucleic acid construct according to any one of [1] to [6], comprising a poly-A addition signal between the mutant gene sequence of (2) and the sequence of (3).
[8] The nucleic acid construct according to any one of [1] to [7], wherein the cleavage site is a restriction enzyme recognition site.
[9] The nucleic acid construct according to any one of [1] to [8], wherein the nucleic acid construct is a circular nucleic acid construct comprising (2) and (3) in this order downstream of (1), or a linear nucleic acid construct prepared by cleaving the circular nucleic acid construct at the cleavage site; or a linear nucleic acid construct comprising (2) and (3) in this order downstream of (1).
[10] The nucleic acid construct according to any one of [1] to [8], wherein the nucleic acid construct is a circular nucleic acid construct comprising (2) downstream of (1), and comprising (3) upstream of (1), or a linear nucleic acid construct prepared by cleaving the circular nucleic acid construct at the cleavage site; or a linear nucleic acid construct comprising (2) downstream of (1), and comprising (3) upstream of (1).
[11] The nucleic acid construct according to any one of [1] to [10], wherein the gene sequence is a gene sequence encoding a protein that acts to decrease cell survival rate of or a protein whose intracellular expression is detectable.
[12] The nucleic acid construct according to [11], wherein the gene sequence is a sequence of a suicide gene, a DNA damage-inducing gene, a DNA repair-inhibiting gene, a luminescent enzyme gene, a fluorescent protein gene, a cell surface antigen gene, a secretory protein gene, or a membrane protein gene.
[13] A linear nucleic acid construct comprising the following (a) to (d) in the order of (c), (d), (a), and (b) from the upstream side toward the downstream side:
   (a) a promoter region;
   (b) an upstream-side region of a gene sequence encoding a protein;
   (c) a downstream-side region of the gene sequence; and
   (d) a base sequence composed of a first homologous region and a second
   homologous region and capable of replacing the upstream-side region and the downstream-side region by homologous recombination, the base sequence being any one of the following (d-1) to (d-3):
   (d-1) a continuous partial sequence in the gene sequence encoding the protein, the continuous partial sequence containing a region that spans the upstream-side region and the downstream-side region;
   (d-2) a base sequence which is homologous to the partial sequence of (d-2) and encodes the same amino acid sequence as that encoded by said partial sequence; and
   (d-3) a continuous partial sequence in a gene sequence encoding a protein whose sequence is 80% or more identical to that of the previously-mentioned protein and which has the same activity as the previously-mentioned protein, the continuous partial sequence being a base sequence homologous to the partial sequence of (d-1).
[14] A measurement reagent for homologous recombination activity, the measurement reagent comprising the nucleic acid construct according to any one of [1] to [13], wherein the gene sequence is a gene sequence encoding a protein whose intracellular expression is detectable.
[15] A measurement kit for homologous recombination activity, the kit comprising the measurement reagent according to [14].
[16] A screening system for an agent that affects homologous recombination activity, the screening system comprising the nucleic acid construct according to any one of [1] to [13].
[17] A diagnostic agent for a homologous recombination-deficient cancer, the agent comprising the nucleic acid construct according to any one of [1] to [13].
[18] A detection agent for homologous recombination-restored cancer cells, the agent comprising the nucleic acid construct according to any one of [1] to [13], wherein the gene sequence is a gene sequence encoding a protein whose intracellular expression is detectable.
[19] The detection agent according to [18], wherein the homologous recombination-restored cancer cells are PARP inhibitor-resistant cancer cells.
[20] A companion diagnostic agent for predicting an effect of an anticancer drug on homologous recombination-deficient cancer, the agent comprising the nucleic acid construct according to any one of [1] to [13].
[21] The companion diagnostic agent according to [20], wherein the anticancer drug is a DNA-damaging anticancer drug.
[22] The companion diagnostic agent according to [21], wherein the DNA-damaging anticancer drug is a PARP inhibitor.
[23] A therapeutic agent for homologous recombination-restored cancer, the agent comprising the nucleic acid construct according to any one of [1] to [13], wherein the gene sequence is a gene sequence encoding a protein that acts to decrease cell survival rate.
[24] The therapeutic agent according to [23], wherein the homologous recombination-restored cancer is PARP inhibitor-resistant cancer.
[25] A method of measuring homologous recombination activity in test cells, the method comprising:
   introducing a nucleic acid construct according to any one of [1] to [13], wherein the gene sequence is a gene sequence encoding a protein whose intracellular expression is detectable, into test cells whose homologous recombination activity is to be measured, and into HR-proficient control cells having normal homologous recombination activity;
   measuring the expression levels of the protein in the test cells and the HR-proficient control cells; and
   comparing the expression level in the test cells with the expression level in the HR-proficient control cells;
   wherein a protein expression in the test cells that is lower than the protein expression in the HR-proficient control cells indicates that the test cells have a lower homologous recombination activity.
[26] A method of identifying a candidate of an agent that affects homologous recombination activity, the method comprising:
   introducing the nucleic acid construct according to any one of [1] to [13] into cells having normal homologous recombination activity, and then treating the cells with each compound in a compound group; or treating cells having normal homologous recombination activity with each compound in a compound group, and then introducing the nucleic acid construct according to any one of [1] to [13] into the cells; and
   measuring expression of the protein.
[27] The method according to [26], wherein the protein is a protein whose intracellular expression is detectable as a signal, the method comprising selecting a compound as a candidate of an inhibitor that inhibits homologous recombination activity when the signal of the protein detected is lower in the cells treated with the compound than in the cells not treated with the compound, or selecting a compound as a candidate of an enhancer that promotes homologous recombination activity when the signal of the protein rises more rapidly or a higher signal is detected in the cells treated with the compound compared to the cells not treated with the compound.
[28] The method according to [26], wherein the protein is a protein that acts to decrease cell survival rate, the method comprising selecting a compound as a candidate of an inhibitor that inhibits homologous recombination activity when the decrease in the cell survival rate is suppressed in the cells treated with the compound compared to the cells not treated with the compound, or selecting a compound as a candidate of an enhancer that promotes homologous recombination activity when the decrease in the cell survival rate occurs earlier in the cells treated with the compound than in the cells not treated with the compound.
[29] A diagnostic method for homologous recombination-deficient cancer, the method comprising:
   introducing the nucleic acid construct according to any one of [1] to [13] into cancer cells of a cancer patient; and
   measuring expression of the protein.
[30] The method according to [29], wherein the cancer cells are cells separated from the cancer patient, and the introduction of the nucleic acid construct into the cancer cells is carried out in vitro.
[31] The method according to [29],
   wherein:
   the protein is a protein whose intracellular expression is detectable as a signal;
   the introduction of the nucleic acid construct into the cancer cells is carried out by administering the nucleic acid construct to the cancer patient; and
   whether or not the signal of the protein is detected from a cancer lesion is investigated.
[32] The method according to [29],
   wherein:
   the protein is a secretory protein whose intracellular expression is detectable;
   the introduction of the nucleic acid construct into the cancer cells is carried out by administering the nucleic acid construct to the cancer patient; and
   the activity of the protein in blood separated from the patient after the administration of the nucleic acid construct is measured.
[33] A detection method for homologous recombination-restored cancer cells, the method comprising:
   introducing the nucleic acid construct according to any one of [1] to [13] into cancer cells of a cancer patient that is a patient under treatment with a PARP inhibitor or that is a patient who was once diagnosed with homologous recombination-deficient cancer; and
   measuring expression of the protein.
[34] The method according to [33], wherein the cancer cells are cells separated from the patient, and the introduction of the nucleic acid construct into the cancer cells is carried out in vitro.
[35] The method according to [33],
   wherein:
   the protein is a protein whose intracellular expression is detectable as a signal;
   the introduction of the nucleic acid construct into the cancer cells is carried out by administering the nucleic acid construct to the patient; and
   whether or not the signal of the protein is detected from a cancer lesion is investigated.
[36] A method of predicting an effect of an anticancer drug on homologous recombination-deficient cancer, the method comprising:
   introducing the nucleic acid construct according to any one of [1] to [13] into cancer cells of a cancer patient; and
   measuring expression of the protein.
[37] The method according to [36], wherein the anticancer drug is a DNA-damaging anticancer drug.
[38] The method according to [37], wherein the DNA-damaging anticancer drug is a PARP inhibitor.
[39] The method according to any one of [36] to [38], wherein the cancer cells are cells separated from the patient, and the introduction of the nucleic acid construct into the cancer cells is carried out in vitro.
[40] The method according to any one of [36] to [38],
   wherein:
   the protein is a protein whose intracellular expression is detectable as a signal;
   the introduction of the nucleic acid construct into the cancer cells is carried out by administering the nucleic acid construct to the patient; and
   whether or not the signal of the protein is detected from a cancer lesion is investigated.
[41] The method according to any one of [36] to [38],
   wherein:
   the protein is a secretory protein whose intracellular expression is detectable;
   the introduction of the nucleic acid construct into the cancer cells is carried out by administering the nucleic acid construct to the cancer patient; and
   activity of the protein in blood separated from the patient after the administration of the nucleic acid construct is measured.
[42] A therapeutic method for homologous recombination-restored cancer, the method comprising administering a nucleic acid construct according to any one of [1] to [13], wherein the gene sequence is a gene sequence encoding a protein that acts to decrease cell survival rate, to a patient having the homologous recombination-restored cancer.
[43] The method according to [42], wherein the nucleic acid construct is topically administered into a tumor or into the vicinity of a tumor of the patient.
[44] The method according to [42] or [43], wherein the homologous recombination-restored cancer is PARP inhibitor-resistant cancer.
[45] A method of predicting whether or not a gene mutation identified in a cancer patient is a pathogenic mutation that deteriorates homologous recombination activity, the method comprising:
   constructing an expression vector that expresses a mutant gene having the same mutation as the previously-mentioned mutation;
   providing an expression vector that expresses a wild-type gene not having the mutation;
   introducing each of the mutant-gene expression vector and the wild-type-gene expression vector, and also introducing the nucleic acid construct according to any one of claims 1 to 11, into cells deficient in the gene; and
   measuring expression of the protein in the cells in which each vector and the nucleic acid construct are introduced;
   wherein the mutation is indicated to be a pathogenic mutation that deteriorates homologous recombination activity when the expression level of the protein in the cells in which the mutant-gene expression vector is introduced is lower than the expression level of the protein in the cells in which the wild-type-gene expression vector is introduced.

### EFFECT OF THE INVENTION

By the present invention, a novel means that enables evaluation of the homologous recombination activity itself of a cell as an absolute value rather than relative evaluation is provided. By the nucleic acid construct of the present invention, the presence or absence of homologous recombination activity can be detected using a transient expression system in a very short time. The nucleic acid construct of the present invention is useful as a measurement reagent or measurement kit for homologous recombination activity, and can also be used, for example, as a screening system for an agent that affects homologous recombination activity, as a diagnostic agent for a homologous recombination-deficient cancer, as a detection agent for homologous recombination-restored cancer cells, or as a companion diagnostic agent for predicting an effect of an anticancer drug on a homologous recombination-deficient cancer. Further, by utilizing a sequence of a gene encoding a protein that acts to decrease the cell survival rate such as a suicide gene as the gene sequence, a nucleic acid construct that selectively exerts a cytocidal effect on cells having homologous recombination activity can be obtained. Anticancer-drugresistant cancers with restored homologous recombination activity have been a serious problem in the treatment with a PARP inhibitor, and no therapeutic method for such cancers exists at present. However, the nucleic acid construct of the present invention enables treatment of even such resistant cancers. Further, by utilization of the construct of the present invention, whether or not a mutation identified in a cancer patient is a pathogenic mutation can be determined or predicted.

### BRIEF DESCRIPTION OF THE DRAWINGS

Fig. 1 is a schematic diagram illustrating nucleic acid constructs (version 1) of the present invention prepared in Examples.
Fig. 2 is a structural drawing of pIRES used for the preparation of plasmid-vector-type nucleic acid constructs in Examples (the structural drawing in pIRES Vector Information of Clontech was partially modified).
Fig. 3 is an example of the result of a luciferase assay using a nucleic acid construct prepared using a luciferase gene (DR-Nluc construct, version 1).
Fig. 4 is an example of the result of investigation of the cytocidal effect of a nucleic acid construct prepared using the suicide gene DT-A (DR-DTA construct, version 1), which investigation was carried out by the colony formation method.
Fig. 5 is an example of the result of investigation of the cytocidal effect of a DR-DTA construct of version 1 by a growth inhibition test.
Fig. 6 is an example of the result of a luciferase assay performed in HT1080 cells, MDA-MB-436 cells, or HCC1937 cells into which a DR-Nluc construct of version 1 was introduced.
Fig. 7 is a diagram illustrating the difference between version 1 and 2 DR-Nluc constructs.
Fig. 8 is an example of the result of a luciferase assay using a DR-Nluc-v2 construct.
Fig. 9 is an example of the result of investigation of the cytocidal effect of a DR-DTA-v2 construct by the colony formation method.
Fig. 10 is a diagram illustrating the structure of a DR-TK construct prepared using the suicide gene HSV-TK.
Fig. 11 is an example of the result of investigation of the cytocidal effect of a DR-TK construct by a growth inhibition test.
Fig. 12 is an example of the result of a luciferase assay using a DR-SecNluc construct.

### MODE FOR CARRYING OUT THE INVENTION

A nucleic acid construct of a first mode of the present invention comprises the following (1) to (3):
(1) a promoter region;
(2) a mutant gene sequence containing a cleavage site in a gene sequence encoding a protein; and
(3) a base sequence according to any one of the following (i) to (iii), the base sequence being composed of a first homologous region and a second homologous region and capable of replacing, by homologous recombination, a partial region in the mutant gene sequence of (2), the partial region containing the cleavage site:
   (i) a continuous partial sequence in the gene sequence encoding the protein, the continuous partial sequence containing upstream and downstream regions adjacent to the cleavage site in the mutant gene sequence of (2);
   (ii) a sequence which is homologous to the partial sequence of (i) and encodes the same amino acid sequence as that encoded by said partial sequence; and
   (iii) a continuous partial sequence in a gene sequence encoding a protein whose sequence is 80% or more identical to that of the previously-mentioned protein and which has the same activity as the previously-mentioned protein, the continuous partial sequence being a sequence homologous to the partial sequence of (i).

The promoter of (1) is not limited, and may be any promoter as long as a promoter activity can be exerted in a cell (typically a mammalian cell such as a human cell). In general, a promoter that constitutively exerts a strong promoter activity in the cell into which the nucleic acid construct of the present invention is introduced may be preferably used. Or, an inducible promoter that exerts the promoter activity under certain conditions may be used. In the following Examples, a human cytomegalovirus promoter that constitutively exerts a strong promoter activity in a cell was used. However, the promoter is not limited thereto. Further, the promoter is not limited to a virus-derived promoter, and the origin of the promoter is not limited as long as it is a sequence having a promoter activity in a cell, preferably in a human cell.

The gene sequence employed for the nucleic acid construct of the present invention is not limited as long as it is a gene sequence encoding a protein. In the present description, the protein encoded by this gene sequence may be referred to as protein A. In the first mode of the nucleic acid construct of the present invention, in a cell having HR activity, a partial region containing the cleavage site in the mutant gene sequence of (2) (in cases where the stop codon is contained upstream of the cleavage site in the mutant sequence of (2), a stop codon and the cleavage site) is replaced by the sequence of (3) by HR to reconstruct the gene sequence encoding protein A, resulting in production of protein A. On the other hand, in a cell having no HR activity, the replacement of the cleavage site (the stop codon and the cleavage site in the cases where the stop codon is contained) in the mutant gene sequence of (2) by the sequence of (3) does not occur, so that protein A is not expressed. Thus, whether or not the cell to which the nucleic acid construct has been introduced has HR activity can be detected based on the amount of expression of protein A, preferably based on the expression level of the activity of protein A, or HR activityrestored cancer cells can be selectively killed utilizing the activity of protein A. The gene sequence encoding protein A reconstructed by the homologous recombination may be a sequence containing the entire coding region of a naturally occurring gene, or may be a sequence composed of part thereof or a modified sequence thereof. For example, only a region encoding a domain required for the exertion of the activity of the protein (for example, a sequence encoding a protein fragment obtained by removal of domains that are not necessary for the protein activity itself such as a domain required for membrane localization) of a naturally occurring gene sequence may be used as the gene sequence in the present invention. Further, for fluorescent proteins and luminescent proteins, genes with various modifications have been widely commercially utilized. Such gene sequences encoding non-naturally occurring proteins may also be used.

Protein A is preferably a protein whose expression level can be rapidly and simply measured. For example, the protein used as protein A is preferably a protein whose activity can be measured directly, or indirectly using, for example, a phenotypic change that occurs in the cells due to the activity of the protein, rather than a protein requiring measurement of the amount of production or accumulation of the protein itself. Examples of such a protein include proteins whose intracellular expression is detectable based on a signal such as luminescence due to reaction with a substrate, or fluorescence of the protein itself. Another example is a protein that acts to decrease the cell survival rate. In this case, the activity of the protein can be indirectly measured as a decrease in the cell survival rate.

The term "measurement of expression (the expression level) of protein A" includes measurement of the amount of production or accumulation of protein A, and measurement of the activity of protein A. As described above, in the present invention, it is preferred to measure the expression of protein A by directly or indirectly measuring the activity of protein A. In other words, it is preferred to employ, as protein A, a protein whose activity can be directly or indirectly measured.

Preferred examples of protein A include a protein having an action to decrease the cell survival rate, and a protein whose intracellular expression is detectable.

Specific examples of the gene encoding a protein having an action to decrease the cell survival rate include, but are not limited to, suicide genes, DNA damage-inducing genes, and DNA repair-inhibiting genes.

Examples of the suicide genes include genes encoding a protein which by itself is toxic or injurious to cells, genes encoding a protein which acts on another compound to produce a toxic substance, and genes encoding a naturally occurring or non-naturally occurring protein that inhibits an endogenous protein to exert cytotoxicity by the dominant negative effect. Specific examples of the suicide genes include the diphtheria toxin A fragment gene (*DT-A*) (the DT-A protein itself has toxicity to cells, and kills the cells), the herpesvirus-derived thymidine kinase gene (*HSV-tk*) (HSV-tk protein acts on ganciclovir, 5-iodo-2'-fluoro-2'deoxy-1-beta-D-arabino-furanosyl-uracil (FIAU), or the like to convert it to a toxic substance having a DNA synthesis-inhibiting activity; use of this gene as a suicide gene requires treatment of the cells with ganciclovir, FIAU, or the like), and the p53 gene (its overexpression induces cell cycle arrest or apoptosis to cause cell death). Further specific examples of the suicide genes include naturally occurring nucleases represented by restriction enzymes and meganucleases; and artificial nucleases represented by ZFN and TALEN, and the CRISPR system (these are also included in specific examples of the DNA damage-inducing genes). However, suicide genes are not limited to these specific examples.

Examples of the gene encoding a protein whose intracellular expression is detectable include, but are not limited to, luminescent enzyme genes such as luciferase genes (including genes encoding a secretory luciferase); fluorescent protein genes (genes encoding a naturally occurring or an artificial fluorescent protein including GFP, CFP, OFP, RFP, and YFP, and modified types thereof); cell surface antigen genes; secretory protein genes; and membrane protein genes. Luminescent enzyme genes and fluorescent protein genes are genes encoding a protein whose intracellular expression is detectable as a signal such as luminescence or fluorescence, and such genes are included in examples of genes that may be preferably used in the present invention.

The sequence of (2) is a mutant gene sequence having a structure in which a mutation (more specifically, a cleavage site) is introduced within a gene sequence encoding protein A. In cases where the gene sequence encoding protein A employed for the sequence of (2) is a gene sequence derived from a eukaryotic animal, a base sequence of mRNA from which an intron(s) is/are removed may be used. As long as the gene sequence encodes protein A, the gene sequence may be a naturally occurring gene sequence, or may be a sequence comprising, at a site(s) other than the site where the mutation is introduced, one or more bases that are different from those in the naturally occurring gene sequence (typically a silent mutation(s)).

A stop codon may be contained upstream of the cleavage site. In this case, the stop codon and the cleavage site are introduced into the gene sequence encoding protein A such that they are arranged in the order of [stop codon] - [cleavage site] from the 5'-side. The inclusion of the stop codon upstream of the cleavage site is not indispensable. However, in cases where the nucleic acid construct of the first mode is prepared as a circular construct and used (introduced into cells, or administered to a patient) as it is in the circular shape without linearization by cleavage at the cleavage site, a stop codon is preferably placed upstream of the cleavage site in order to securely stop expression of active protein A from the gene sequence of (2) in a cell deficient for homologous recombination activity. The stop codon may be directly linked to the cleavage site, or a certain base sequence may be present therebetween. The strand length of [stop codon] - [cleavage site] is preferably not more than about 50 bases, for example, not more than about 30 bases.

A typical example of the cleavage site is a restriction enzyme recognition site. However, the cleavage site is not limited thereto. The cleavage may be carried out not only by cleavage by a restriction enzyme, but also by, for example, use of a genome editing technique that causes a DNA strand break. Specific examples of such a cleavage include cleavage by a CRISPR/Cas system, i.e., cleavage by a complex of a guide RNA and a Cas protein such as Cas9. In cases where the complex is used, a PAM sequence is introduced into an appropriate position such that the cleavage by the complex occurs at an appropriate position downstream of the stop codon. In cases of *Streptococcus pyogenes* type II-derived Cas9, which is the most commonly used system among the known CRISPR/Cas systems, the PAM sequence is 5'-NGG (N represents A, T, G, or C). By the complex, the nucleic acid construct is cleaved several bases upstream of the PAM sequence. In the case of the cleavage by the CRISPR/Cas system, the PAM sequence and the actual position of the cleavage located several bases upstream thereof constitute the cleavage site in the present invention. (3) of the nucleic acid construct of the first mode has the above-described structure, and does not contain the cleavage site. Therefore, of course, (3) is not cleaved by the treatment for cleaving the nucleic acid construct at the cleavage site.

In the present invention, the strand length of a nucleic acid is expressed using the unit "bases". The "bases" means "base pairs" in cases where the nucleic acid is a double-stranded nucleic acid. [Cleavage site], or [stop codon] - [cleavage site] may be inserted into the gene sequence encoding protein A, or [cleavage site], or [stop codon] - [cleavage site] may be introduced such that part of the gene sequence encoding protein A is replaced therewith. The pCMV-DR-DTA constructs (versions 1 and 2), the pCMV-DR-Nluc constructs (versions 1 and 2), and the pCMV-DR-TK construct that were constructed in the following Examples are examples of the latter case.

In the mutant gene sequence of (2), the upstream side and downstream side of [cleavage site] (when the stop codon is contained, [stop codon] - [cleavage site]) are hereinafter referred to as 5'-side region and 3'-side region, respectively. In cases where the construct is cleaved at the cleavage site and used in the linearized state, in a cell in which HR does not occur, a protein A fragment which lacks the region positioned downstream of the cleavage site is produced. In cases where the circular construct is used as it is, in a cell in which HR does not occur, translation stops by the introduced stop codon to produce a protein A fragment composed of the 5'-side region. Thus, the position of [cleavage site] or [stop codon] - [cleavage site] in the mutant gene sequence should be determined taking into account the size of the 5'-side region such that the activity of protein A is not exerted by the 5'-side-region fragment. The size of the 5'-side region is not limited, and may be appropriately set depending on the type of protein A. In general, the size of the 5'-side region may be not more than half, for example, not more than 40/100, not more than 30/100, or not more than 25/100 of the entire length of protein A. Although there is no lower limit of the size of the 5'-side region, the size is usually set to not less than 50 bases.

The sequence of (3) is a base sequence composed of a first homologous region and a second homologous region and capable of replacing, by homologous recombination, a partial region in the mutant gene sequence of (2), the partial region containing the cleavage site, and is any one of the following (i) to (iii):
(i) a continuous partial sequence in the gene sequence encoding protein A, the continuous partial sequence containing upstream and downstream regions adjacent to the cleavage site in the mutant gene sequence of (2);
(ii) a base sequence which is homologous to the partial sequence of (i) and encodes the same amino acid sequence as that encoded by said partial sequence of (i); and
(iii) a continuous partial sequence in a gene sequence encoding a protein whose sequence is 80% or more identical to that of protein A and which has the same activity as protein A, the continuous partial sequence being a base sequence homologous to the partial sequence of (i).

The entire 5'-side region in the mutant gene sequence of (2), or a 3'-end-side portion of this region (that is, at least a 3'-end-side portion of the 5'-side region) corresponds to the upstream region adjacent to the cleavage site (or the stop codon and the cleavage site) in the mutant gene sequence of (2) (hereinafter referred to as "adjacent upstream region" for convenience) in the sequence of (i) of (3). In the sequence of (i), the adjacent upstream region constitutes the first homologous region. The at least a 3'-end-side portion of the 5'-side region in the mutant gene sequence of (2), and the adjacent upstream region of (3)(i), are referred to as "first homologous region set" for convenience. The entire 3'-side region or a 5'-end-side portion of this region (that is, at least a 5'-end-side portion of the 3'-side region) corresponds to the downstream region adjacent to the cleavage site (or the stop codon and the cleavage site) in the mutant gene sequence of (2) ("adjacent downstream region") in the sequence of (i) of (3). In the sequence of (i), the adjacent downstream region constitutes the second homologous region. The at least a 5'-end-side portion of the 3'-side region in the mutant gene sequence of (2), and the adjacent downstream region of (3)(i), are referred to as "second homologous region set" for convenience. The strand length of each homologous region may be at least 20 bases, or may be, for example, not less than 50 bases, not less than 60 bases, not less than 70 bases, or not less than 80 bases. Although there is no upper limit, the strand length is usually not more than 10,000 bases, or may be, for example, not more than 5,000 bases, not more than 1,000 bases, not more than 500 bases, or not more than 300 bases.

The (i) of (3) is a mode in which the homology in the first homologous region set and the homology in the second homologous region set are 100%, and in which the corresponding sequences are completely the same.

The (ii) of (3) is a mode in which the encoded amino acid sequence is the same as (a partial sequence of) the amino acid sequence of protein A, but in which at least one of the homology in the first homologous region set and the homology in the second homologous region set is less than 100%. It is widely known in the art that, even in cases where corresponding sequences are not completely the same, homologous recombination occurs as long as there is not less than a certain level of homology. The sequence of (ii) is preferably 90% or more homologous, more preferably 95% or more, or 98% or more homologous, to the partial sequence of (i). For example, the homology in the first homologous region set is preferably not less than 90%, not less than 95%, or not less than 98%, and the homology in the second homologous region set is preferably not less than 90%, not less than 95%, or not less than 98%. In the sequence of (ii), of course, the regions corresponding to the adjacent upstream region and adjacent downstream region in (i) constitute the first homologous region and the second homologous region, respectively.

The (iii) of (3) is a continuous partial sequence in a gene sequence encoding a protein whose sequence is 80% or more, for example, 85% or more, 90% or more, 95% or more, or 98% or more, identical to that of protein A, and which has the same activity as protein A, the continuous partial sequence being a base sequence homologous to the partial sequence of (i). This is a mode in which at least one of the homology in the first homologous region set and the homology in the second homologous region set is less than 100%, and in which the encoded amino acid sequence is not the same as the amino acid sequence of protein A. Even if the encoded amino acid sequence is partially different from the amino acid sequence of protein A, the base sequence can be employed as the sequence of (3) as long as the encoded protein has the activity of protein A. Preferably, (iii) is 90% or more, for example, 95% or more, or 98% or more, homologous to the partial sequence of (i), and the encoded amino acid sequence is 80% or more, for example, 85% or more, 90% or more, 95% or more, or 98% or more, identical to the amino acid sequence encoded by the partial sequence of (i).

Conservative substitution, that is, substitution to an amino acid having similar chemical properties, is unlikely to cause deterioration of properties and activity of a protein. Amino acids having similar side chains have similar chemical properties. Based on the similarities among the side chains, amino acids can be classified into, for example, a group of amino acids having an aliphatic side chain (glycine, alanine, valine, leucine, and isoleucine), a group of amino acids having an aliphatic hydroxyl side chain (serine and threonine), a group of amino acids having an amide-containing side chain (asparagine and glutamine), a group of amino acids having an aromatic side chain (phenylalanine, tyrosine, and tryptophan), a group of amino acids having a basic side chain (arginine, lysine, and histidine), a group of amino acids having an acidic side chain (aspartic acid and glutamic acid), and a group of amino acids having a sulfur-containing side chain (cysteine and methionine). Substitution to another amino acid belonging to the same group is conservative substitution. Typical examples of the amino acid sequence encoded by the base sequence of (iii) include, but are not limited to, an amino acid sequence in which a conservative substitution(s) is/are introduced in the amino acid sequence encoded by the partial sequence of (i).

The sequences of (2) and (3) used for the nucleic acid construct of the first mode can be prepared by PCR amplification using appropriate primers, from a known plasmid in which a gene encoding protein A is incorporated, or from cultured cells or a cDNA library of an organism species that expresses protein A. A stop codon is required at the 3'-end of the mutant gene sequence of (2). The sequence of (3) is a sequence that functions as a substrate for the replacement of the part of the mutant gene sequence of (2) by homologous recombination, and is a region not translated before the homologous recombination occurs. Therefore, it is not necessary to add a stop codon to the 3'-end of the sequence of (3). The following Examples describe cases where a stop codon is introduced at the 3'-end of an iNluc fragment or an iDTA fragment, which corresponds to the sequence of (3). Although a stop codon may be introduced as in these cases, the introduction is not essentially required. When desired, in the PCR amplification, in addition to the stop codon, an appropriate adaptor sequence(s) used for cloning of the nucleic acid construct of the present invention and/or the like may be introduced to the two gene fragments. The sequence of (2) comprising, at a site(s) other than the site where the mutation is introduced, one or more bases that are different from those in the naturally occurring gene sequence, and the sequences of (ii) and (iii) of (3), can be prepared by a method such as in vitro mutagenesis or artificial gene synthesis. The poly-A addition signal may be placed downstream of the mutant gene sequence of (2) (between the mutant gene sequence of (2) and the sequence of (3) in a case where the sequence of (3) is placed downstream of the mutant gene sequence of (2)). However, in the nucleic acid construct of the present invention, the poly-A addition signal is not an indispensable element. Since mRNAs that appropriately function without a poly-A, such as a histone mRNA, are known, the poly-A addition signal may be omitted also in the nucleic acid construct of the present invention.

The nucleic acid construct of the first mode may be a construct in which (1) to (3) are incorporated in a plasmid vector, may be a construct in which (1) to (3) are incorporated in a virus vector, or may be in the form of a nucleic acid fragment in which they are not incorporated in a vector. As described above, a poly-A addition signal may be placed downstream of (2) (between (2) and (3) in a case where (3) is placed downstream of (2)). In the case of either a plasmid vector or a virus vector, the order of arrangement from the 5'-side toward the downstream side in the vector is [promoter region] - [mutant gene sequence of (2)] - [poly-A addition signal] - [sequence of (3)], or [sequence of (3)] - [promoter region] - [mutant gene sequence of (2)] - [poly-A addition signal]. Also in the case of a nucleic acid fragment not incorporated in a vector, the arrangement from the 5'-side is the same as described above. In cases of use as a pharmaceutical such as a therapeutic agent or diagnostic agent, or as a reagent such as a measurement reagent, the nucleic acid construct of the first mode using a plasmid vector may be used as it is in the form of a circular nucleic acid construct containing (2) and (3) in this order downstream of the promoter region, or in the form of a circular nucleic acid construct containing (3) upstream of, and containing (2) downstream of, the promoter region. Or, the circular nucleic acid construct may be cleaved at the cleavage site arranged inside the mutant gene sequence of (2), and may be used in the linear form. The nucleic acid construct using a virus vector may be a linear nucleic acid construct containing (2) and (3) in this order downstream of the promoter region, or containing (3) upstream of, and containing (2) downstream of, the promoter region. This linear nucleic acid construct also may be used either without cleavage or after cleavage at the cleavage site. The nucleic acid construct in the form of a nucleic acid fragment not incorporated in a vector is usually linear, and contains (2) and (3) in this order downstream of the promoter region, or contains (3) upstream of, and (2) downstream of, the promoter region. The nucleic acid construct of the present invention in the form of a construct incorporated in a vector may contain, in addition to (1) to (3), common elements such as an origin of replication, a translation initiation site, or a selection marker gene including a drug resistance gene, depending on the type of the vector.

The second mode of the nucleic acid construct of the present invention is a linear nucleic acid construct comprising the following (a) to (d) in the order of (c), (d), (a), and (b) from the upstream side toward the downstream side:
(a) a promoter region;
(b) an upstream-side region of a gene sequence encoding a protein;
(c) a downstream-side region of the gene sequence; and
(d) a base sequence composed of a first homologous region and a second homologous region and capable of replacing the upstream-side region and the downstream-side region by homologous recombination, the base sequence being any one of the following (d-1) to (d-3):
   (d-1) a continuous partial sequence in the gene sequence encoding the protein, the continuous partial sequence containing a region that spans the upstream-side region and the downstream-side region;
   (d-2) a base sequence which is homologous to the partial sequence of (d-1) and encodes the same amino acid sequence as that encoded by said partial sequence; and
   (d-3) a continuous partial sequence in a gene sequence encoding a protein whose sequence is 80% or more identical to that of the previously-mentioned protein and which has the same activity as the previously-mentioned protein, the continuous partial sequence being a base sequence homologous to the partial sequence of (d-1).

(b) and (c) are elements corresponding to the element (2) in the first mode, and constituted by the upstream-side region and the downstream-side region, respectively, of a gene sequence encoding the same protein (protein A). They may be constituted by fragments obtained by dividing the entire gene sequence into two parts. Or, they may be constituted by an upstream-side partial region containing the 5'-end and a downstream-side partial region containing the 3'-end, in neither of which an internal partial region of the gene sequence is contained. In the latter case, a base sequence generated by linking the base sequences of (b) and (c) together is not identical to the full-length gene sequence encoding protein A, and is shorter than the full-length gene sequence due to lack of the internal partial region.

(d) is an element corresponding to (3) in the first mode, and (d-1) to (d-3) correspond to (i) to (iii) of (3), respectively. In cases where (b) and (c) are constituted by fragments obtained by dividing the full-length gene sequence into two parts, (d-1) is a partial sequence of the gene sequence, which partial sequence contains the division site, and the regions upstream of and downstream of the division site constitute the first homologous region and the second homologous region, respectively. In cases where (b) and (c) are constituted by an upstream-side partial region containing the 5'-end and a downstream-side partial region containing the 3'-end, in neither of which an internal partial region of the gene sequence is contained, (d-1) is a partial sequence of the gene sequence, which partial sequence contains the internal partial region, and the upstream and downstream regions adjacent to the internal partial region constitute the first homologous region and the second homologous region, respectively.

The nucleic acid construct of the second mode also may or may not contain a poly-A addition signal. When a poly-A addition signal is represented as (e), and when (e) is contained, the elements may be contained in the order of (c), (e), (d), (a), and (b) from the upstream side toward the downstream side.

The above-described matters are the differences from the first mode, and other conditions (for example, the protein A-encoding gene sequence, protein A, the promoter region of (a), preferred examples of (d-2) and (d-3), and the size of the homologous region) are the same as those of the nucleic acid construct of the first mode.

In the second mode of the nucleic acid construct, in a cell having HR activity, at least part of the 3'-end side of (b) and at least part of the 5'-end side of (c) are replaced by (d) by HR to reconstruct the gene sequence encoding protein A, resulting in production of protein A. On the other hand, in a cell having no HR activity, the replacement by (d) does not occur, so that protein A is not expressed. Thus, the nucleic acid construct of the second mode can be used in a similar manner to the first mode.

When the term "nucleic acid construct of the present invention" is simply used in the present description hereinbelow, it includes the first mode and the second mode unless otherwise specified.

The nucleic acid construct of the present invention may be either DNA or RNA. In general, DNA is preferred in cases of a plasmid-vector-type nucleic acid construct. Typical examples of the virus-vector-type nucleic acid construct include a nucleic acid construct that is a double-stranded DNA in the form of a virus expression plasmid. Examples of the virus-vector-type nucleic acid construct also include a virus expressed from a virus expression plasmid. The nucleic acid construct that is a virus expressed from the virus expression plasmid is RNA in cases where the vector is an RNA virus vector, and DNA in cases where the vector is a DNA virus vector. In cases of a nucleic acid construct in a form in which it is not incorporated in a vector, the nucleic acid construct is usually DNA. The nucleic acid construct of the present invention may comprise a nucleotide analog in part thereof. Examples of the nucleotide analog include, but are not limited to, bridged nucleic acids such as LNA, ENA, and PNA; and modified bases such as Super T (registered trademark) and Super G (registered trademark).

The nucleic acid construct of the present invention can be used, for example, as a measurement reagent for homologous recombination activity. In this mode of use, as the gene sequence encoding protein A, a gene sequence encoding a protein whose intracellular expression is detectable may be preferably used. The measurement reagent may be a liquid reagent containing an appropriate additive(s) for the stability or the like of the nucleic acid construct, or may be a powder reagent prepared by freeze-drying of the nucleic acid construct. The nucleic acid construct of the present invention may be introduced into test cells whose homologous recombination activity is to be measured, and cells having normal homologous recombination activity or cells not deficient in homologous recombination activity (HR-proficient control cells), and then expression of protein A may be measured, followed by comparing the expression level of protein A (a signal of protein A) between the test cells and the HR-proficient control cells. The introduction of the nucleic acid construct may be transient. Further, cells deficient in homologous recombination activity may be used as HR-deficient control cells.

The HR-proficient control cells are not limited as long as they are cells having normal homologous recombination activity. Any of a number of known wild-type mammalian cell lines having no mutation in HR-related genes (such as *ATM*, *CDK1*, *CDK12*, *MRE11*, *NBS1*, *Rad50, PLK1*, *RNF1*, *RNF8, RNF168*, *ARID1A*, *SMARCAD1*, *CHD1*, *CHD4, LEDGF, RBBP8* (*CtIP*), *BLM, Exol, DNA2, BRCA1*, *BRCA2, PALB2, BARD1, BRIP1*, *BAP1*, *Rad51B*, *Rad51C*, *Rad51D, Rad51AP1*, *Rad52, XRCC2, XRCC3, Rad51, Rad54L (Rad54), Rad54B, FBH1*, *WRN, SMARCAL1*, *ATRX, PTEN, MUS81*, *EME1*, *EME2, SLX1*, *SLX4, XPF, ERCC1*, *RPA1*, *RPA2, PRA3, FANCA, FANCB, FANCC, FANCD2, FANCE, FANCF, FANCG*, *FANCI, FANCL, FANCM, LIG1, LIG3, POLD, HROB, MCM8, MCM9, HELQ, RAD21*, and *RAD21L1*) may be preferably used as the HR-proficient control cells. Specific examples of known mammalian cell lines having normal homologous recombination activity include, but are not limited to, human cell lines such as Nalm-6 (a human pre-B-cell leukemia cell-derived cell line) and HT1080 (a human fibrosarcoma cell line), which are also used in the Examples below, U2OS (a human osteosarcoma-derived cell line), HeLa (a human cervical cancer-derived cell line), HCT116 (a human colon adenocarcinoma-derived cell line), MCF-7 (a human breast adenocarcinoma-derived cell line), HAP1 (a human chronic myelogenous leukemia-derived cell line), HEK293 (a human embryonic kidney-derived cell line), TIG-7 (a human lung-derived cell line), TIG-3 (a human lung-derived cell line), iPS cells (human induced pluripotent stem cells; established from normal human cells), and ES cells (human embryonic stem cells). Cell lines derived from human hereditary breast cancer or hereditary ovarian cancer often have a mutation(s) in the *BRCA1* gene or the *BRCA2* gene, which are HR-related genes, and are hence often deficient in homologous recombination activity. Therefore, they are inappropriate as HR-proficient control cells. Whether or not an arbitrary cell line has normal homologous recombination activity (whether or not the cell line is deficient in homologous recombination activity) can be investigated by using the nucleic acid construct of the present invention, or by using a conventionally known technique. In cases where the investigation is carried out using the nucleic acid construct of the present invention, when the cells have homologous recombination activity almost equal to or higher than the above-described specific example of the HR-proficient control cells, the cells may be judged as having normal homologous recombination activity (being not deficient in homologous recombination activity).

Further, in order to provide an additional positive control, a construct in which a normal gene sequence encoding protein A is linked to a promoter under the control of the promoter (positive control construct) may be introduced into the test cells (and, when desired, into the control cells). The lower the homologous recombination activity of the test cells, the lower the expression (a signal of protein A detected from the test cells) of protein A in the test cells. Thus, a signal of protein A in the test cells that is lower than the signal of protein A in the HR-proficient control cells is an index indicating that the test cells have a lower homologous recombination activity.

Examples of the HR-deficient control cells include cell lines derived from homologous recombination-deficient cancers. A variety of kinds of cell lines derived from homologous recombination-deficient cancers having mutations in the HR-related genes described above (for example, human cell lines such as cell lines derived from human hereditary breast cancer or hereditary ovarian cancer wherein the *BRCA1* gene or the *BRCA2* gene has a mutation(s)), and cell lines prepared by introducing a mutation(s) to one or more HR-related genes and methods of preparing such cell lines are known, and any of such cell lines may be used as the HR-deficient control cells. Since the technique for gene knockout has also been established, HR-deficient cells of various animal species can be prepared by knocking out one or more HR-related genes in mammalian cultured cells having normal homologous recombination activity. Specific examples of known homologous recombination-deficient cell lines include human cell lines including: HR-deficient human cell lines derived from Nalm-6 cells (a *Rad54B*-deficient line and a *Rad54*/*Rad54B-double-*deficient line), which are used also in the Examples below; HR-deficient human cell lines available from ATCC (American Type Culture Collection), such as HCC1395 (a human breast cancer-derived cell line, deficient in *BRCA1*), HCC1599 (a human breast cancer-derived cell line, deficient in *BRCA2*), HCC1937 (a human breast cancer-derived cell line, deficient in *BRCA1*), MDA-MB-436 (a human breast cancer-derived cell line, deficient in *BRCA1*), DoTc2-4510 (a human cervical cancer-derived cell line, deficient in *BRCA2*), RL95-2 (a human uterine cancer-derived cell line, deficient in *BRCA2*), AGS (a human gastric cancer-derived cell line, deficient in *CHD1*), and SNU-5 (a human gastric cancer-derived cell line, deficient in *CHD1*); and HR-deficient human cell lines available from Horizon Discovery Ltd., such as genetically modified HAP1 cell lines (human chronic myelogenous leukemia-derived cell lines) (an *ATRX*-deficient line, a *CHDI* -deficient line, a *CHD4*-deficient line, an *EME1*-deficient line, an EME2-deficient line, a *FANCA*-deficient line, a *FANCC-*deficient line, a *FANCE*-deficient line, a *FANCG*-deficient line, a *FANCF-*deficient line, a *LEDGF-*deficient line, a *LIG3-*deficient line, an *MUS81*-deficient line, a *RAD51AP1*-deficient line, a *RAD54L-*deficient line, and an *RNF8*-deficient line), genetically modified HCT116 cells (human colon adenocarcinoma-derived cell lines) (an *ARID1A*-deficient line, a *LIG3-*deficient line, and a *PTEN*-deficient line), and a genetically modified DLD-1 cell line (a human colon adenocarcinoma-derived cell line) (a *BRCA2*-deficient line). Specific examples of cell lines of non-human animals include: HR-deficient mice (frozen embryos) available from The NCI Mouse Repository (*BRCA1*-deficient mice and *BRCA2*-deficient mice); and HR-deficient dog cells Parks (MCM8-deficient) (Sunetra Das et al., Mol Cancer Ther. Author manuscript; available in PMC 2020 Feb 1. Published in final edited form as: Mol Cancer Ther. 2019 Aug; 18(8): 1460-1471. Published online 2019 Jun 7. doi:10.1158/1535-7163.MCT-18-1346). However, as described above, the HR-deficient cells are not limited to these specific examples since HR-deficient cells can be prepared in various animal species by preparing cells in which one or more HR-related genes are knocked out.

The measurement reagent of the present invention can be provided also as a measurement kit for homologous recombination activity by combining the reagent with at least one item selected from, for example, a plasmid for a positive control (such as a plasmid in which a normal protein A-coding sequence is placed downstream of a promoter, which plasmid is capable of constitutively expressing protein A), a plasmid for a negative control (a plasmid in which a gene sequence encoding protein A is not incorporated), HR-proficient control cells, HR-deficient control cells, manufacturer's instructions, an appropriate substrate substance (in a case where protein A is an enzyme), and the like.

The nucleic acid construct of the present invention can be used also as, for example, a screening system for agents that affect homologous recombination activity. In other words, the nucleic acid construct of the present invention can be used for identification of candidates of agents that affect homologous recombination activity. A method of identifying a candidate of an agent that affects homologous recombination activity using the nucleic acid construct of the present invention comprises:
introducing the nucleic acid construct of the present invention into cells having normal homologous recombination activity, and then treating the cells with each compound in a compound group; or treating cells having normal homologous recombination activity with each compound in a compound group, and then introducing the nucleic acid of the present invention into the cells; and
measuring expression of protein A in the nucleic acid construct-introduced cells treated with the compound and in the nucleic acid construct-introduced cells not treated with the compound. As described above, the measurement of expression of protein A is preferably measurement of the activity of protein A. In this mode of use, as the gene sequence encoding protein A, a gene sequence encoding a protein whose intracellular expression is detectable, especially a gene sequence encoding a protein whose intracellular expression is detectable as a signal, may be preferably used, and a gene sequence encoding a protein that acts to decrease the cell survival rate may also be used. After cleaving, when desired, the nucleic acid construct of the present invention at the cleavage site arranged inside the sequence of (2), the construct may be introduced into cells having normal homologous recombination activity (HR-normal cells, usually mammalian cells such as cultured human cells, are preferably used; the details are the same as those of the HR-proficient control cells), and then the cells may be treated with each compound in a compound group such as a compound library (the order of the introduction of the nucleic acid construct and the treatment with each compound may be reversed), followed by measuring the expression of protein A to investigate whether or not a decrease or an increase in the expression level of protein A, preferably the activity level of protein A, has occurred (whether or not a decrease or an increase in the homologous recombination activity has occurred) due to the treatment with the compound. In cases where protein A is a luciferase, a change in the luciferase activity (luminous reaction) may be measured. In cases where protein A is a protein that acts to decrease the cell survival rate, a change in the cell survival rate may be investigated. The evaluation on whether the expression of protein A is decreased or increased can be carried out by introducing the nucleic acid construct into cells having normal homologous recombination activity, measuring the expression of protein A without carrying out the treatment with the compound, and then investigating whether the expression level is increased or decreased relative to this measured value. In addition to the HR-normal cells, HR-deficient cells that are deficient in homologous recombination activity (for example, a *RAD54*/*RAD54B*-double-deficient line, which is also used in the Examples below; the details are the same as those of the HR-deficient control cells) may be used.

In cases where protein A is a protein whose intracellular expression is detectable as a signal, the selection of a compound can be carried out as follows.

When the signal of protein A detected is lower in HR-normal cells treated with a compound than in HR-normal cells not treated with the compound, the compound can be selected as a candidate of an HR inhibitor that inhibits HR activity.

When the signal of protein A rises more rapidly or a higher signal is detected in HR-normal cells treated with a compound than in HR-normal cells not treated with the compound, the compound can be selected as a candidate of an HR enhancer that promotes HR activity.

In cases where protein A is a protein that acts to decrease the cell survival rate, the selection of a compound can be carried out as follows.

When the decrease in the cell survival rate is more suppressed in HR-normal cells treated with a compound than in HR-normal cells not treated with the compound (in other words, when the survival rate of HR-normal cells treated with a compound is higher than the survival rate of HR-survival cells not treated with the compound), the compound can be selected as a candidate of an HR inhibitor that inhibits HR activity.

When the decrease in the cell survival rate occurred earlier in HR-normal cells treated with a compound than in HR cells not treated with the compound, or the survival rate of HR-normal cells treated with a compound is lower than the survival rate of HR cells not treated with the compound, the compound can be selected as a candidate of an ΣIR enhancer that promotes HR activity.

The nucleic acid construct of the present invention can also be used, for example, as a diagnostic agent for homologous recombination-deficient cancer. Deficiency in homologous recombination has been reported in various cancers. More specifically, for example, deficiency in homologous recombination has so far been reported in breast cancer and ovarian cancer (including hereditary breast ovarian cancer), and also in colorectal cancer, endometrial cancer, gastric cancer, esophagus cancer, pancreatic cancer, hepatobiliary cancer, prostate cancer, liver cancer, non-small-cell lung cancer, and small-cell lung cancer (Non-Patent Documents 6 to 9). It is expected that homologous recombination-deficient cancers will be found also in various cancers other than these (including cancers of various animal species including non-human animals) in the future. It has also been reported that molecular targeted cancer therapies using an EGFR inhibitor, a BRAF inhibitor or the like cause decrease in the homologous recombination activity (Science 20 Dec 2019: Vol. 366, Issue 6472, pp 1473-1480, DOI: 1 0.1126/science.aav44 74). Examples of the homologous recombination-deficient cancer in the present invention include various cancers including the specific examples described above.

In cases where the nucleic acid construct of the present invention is used as a diagnostic agent for homologous recombination-deficient cancer, the construct is introduced into cancer cells of a cancer patient, and expression of protein A is measured. As described above, the measurement of expression of protein A is preferably measurement of the activity of protein A. As the gene sequence encoding protein A, a gene sequence encoding a protein whose intracellular expression is detectable, especially a gene sequence encoding a protein whose intracellular expression is detectable as a signal, may be preferably used, and a gene sequence encoding a protein that acts to decrease the cell survival rate may also be used. In cases where a cell is not deficient in homologous recombination, homologous recombination occurs between the sequence of (3) and part of the sequence of (2) to reconstruct a gene sequence capable of expressing protein A, in the cell. This results in expression of protein A. When protein A is a protein whose intracellular expression is detectable as a signal, the signal of protein A is detected. When protein A is a protein that acts to decrease the cell survival rate, the cell survival rate decreases. On the other hand, in cases where a cell is deficient in homologous recombination, the gene sequence capable of expressing protein A is not reconstructed, so that protein A is not expressed. When protein A is a protein whose intracellular expression is detectable as a signal, the signal of protein A is not detected. When protein A is a protein that acts to decrease the cell survival rate, decrease in the cell survival rate does not occur.

In one mode of the diagnostic agent for homologous recombination-deficient cancer, the cancer cells are cells separated from a cancer patient, and the introduction of the nucleic acid construct into the cancer cells is carried out in vitro. Among the modes of use described later, (1) and (3) are specific examples of this mode. In this mode, a protein whose intracellular expression is detectable and a protein that acts to decrease the cell survival rate may be used as protein A, and the former is more preferred. In this mode, the cancer patient includes solid cancer patients and blood cancer patients. Cells in a cancer tissue separated from a cancer patient by biopsy or surgery, or, in cases of leukemia, cancer cells in blood collected from a cancer patient, may be used. If desired, the cancer cells may be concentrated before the introduction of the nucleic acid construct. The introduction of the nucleic acid construct may be transient. Whether or not cancer cells are deficient in homologous recombination activity can be investigated by measuring the homologous recombination activity of the cancer cells in the same manner as the above-described method of using the measurement reagent for homologous recombination activity. As the HR-proficient control cells, not only the above-described cells having normal homologous recombination activity, but also, for example, non-cancerous cells (non-cancerous peripheral blood cells or the like) collected from the same cancer patient may be used. As the HR-deficient control cells, the above-described homologous recombination-deficient cells may be used.

Preferably, in this mode, the nucleic acid construct is introduced into cancer cells separated from a cancer patient, and into control cells (HR-proficient control cells or HR-deficient control cells, or both), and then expression of protein A is compared between the cancer cells and the control cells.

In cases where expression/activity of protein A is detected in the cancer cells, for example, in cases where the expression level or activity level of protein A detected in the cancer cells is higher than the expression level or activity level of protein A in the HR-deficient control cells, the cancer in the cancer patient is indicated not to be a homologous recombination-deficient cancer. In cases where protein A is a protein whose intracellular expression is detectable as a signal, when the signal of protein A is detected in the cancer cells, for example, when the signal of protein A detected in the cancer cells is higher than the signal of protein A in the HR-deficient control cells, the cancer in the cancer patient is indicated not to be a homologous recombination-deficient cancer. In cases where protein A is a protein that acts to decrease the cell survival rate, when the survival rate of the cancer cells decreases, for example, when the survival rate of the cancer cells is lower than the survival rate of the HR-deficient control cells, the cancer in the cancer patient is indicated not to be a homologous recombination-deficient cancer.

In cases where expression/activity of protein A is not detected in the cancer cells, or in cases where the detected expression or activity of protein A is lower than that in the HR-proficient control cells, the cancer in the cancer patient is indicated to be a homologous recombination-deficient cancer. In cases where protein A is a protein whose intracellular expression is detectable as a signal, when the signal of protein A is not detected in the cancer cells, or when the detected signal is lower than the signal of protein A in the HR-proficient control, the cancer in the cancer patient is indicated to be a homologous recombination-deficient cancer. In cases where protein A is a protein that acts to decrease the cell survival rate, when the survival rate of the cancer cells does not decrease, or when the survival rate of the cancer cells is higher than the survival rate of the HR-proficient control cells, the cancer in the cancer patient is indicated to be a homologous recombination-deficient cancer.

In another mode, protein A is a protein whose intracellular expression is detectable as a signal; the introduction of the nucleic acid construct into the cancer cells is carried out by administering the nucleic acid construct to the cancer patient; and whether or not the signal of protein A is detected from a cancer lesion is investigated. Among the modes of use described later, (2) is a specific example of this mode. In this mode, the cancer patient is typically a solid cancer patient. The nucleic acid construct is preferably topically administered into the tumor or into the vicinity of the tumor of the cancer patient. From the viewpoint of detecting the signal of protein A from the cancer lesion in the body of the cancer patient, a protein that generates the signal without a reaction with a substrate, such as a fluorescent protein, is preferably employed. When the signal of protein A is detected from the cancer lesion (for example, when the level of the detected signal is almost the same as that of a non-cancerous site in the vicinity of the tumor), the cancer of the cancer patient is indicated not to be a homologous recombination-deficient cancer. When the signal of protein A is not detected from the cancer lesion (for example, when the signal from the cancer lesion is clearly lower than the signal from a non-cancerous site in the vicinity of the tumor), a possibility that the cancer of the cancer patient is a homologous recombination-deficient cancer is indicated. In this mode, when a patient is diagnosed with a possible homologous recombination-deficient cancer, cancer cells may be collected from the patient, and the nucleic acid construct may be introduced into the cells to confirm that the cancer is a homologous recombination-deficient cancer.

In still another mode, protein A is a secretory protein whose intracellular expression is detectable (preferably as a signal); the introduction of the nucleic acid construct into the cancer cells is carried out by administering the nucleic acid construct to the cancer patient; and the activity of protein A in blood separated from the patient after the administration of the nucleic acid construct is measured. Among the modes of use described later, (4) is a specific example of this mode. In this mode, the cancer patient is typically a solid cancer patient. The nucleic acid construct is preferably topically administered into the tumor or into the vicinity of the tumor of the cancer patient. In cases where the cancer of the patient is not deficient in homologous recombination, secretory protein A is expressed from the protein A gene reconstructed in the cancer cells, and secreted to the outside of the cancer cells. Therefore, the activity of protein A can be detected using a blood sample of the cancer patient. Since the activity of protein A secreted into the blood can be detected in vitro, a protein that generates a signal by reaction with a substrate may also be preferably used. Typical examples of such a protein include secretory luciferases. As the gene sequence encoding the secretory protein such as a secretory luciferase, a known secretory protein-coding sequence may be used as it is, or a gene sequence prepared by adding a sequence encoding a secretory signal to a protein-coding sequence may be used. As a negative control sample, for example, a blood sample collected from the patient before the administration of the nucleic acid construct may be used. Further, as a positive control, for example, a culture supernatant obtained by introducing the nucleic acid construct into a cell line having normal homologous recombination activity (the details are the same as those of the HR-proficient control cells described above) and culturing the resulting cells may be used. When the activity of protein A is detected in blood, the cancer of the cancer patient is indicated not to be a homologous recombination-deficient cancer. When the activity of protein A is not detected in blood, a possibility that the cancer of the patient is a homologous recombination-deficient cancer is indicated. In this mode, when a patient is diagnosed with a possible homologous recombination-deficient cancer, cancer cells may be collected from the patient, and the nucleic acid construct may be introduced into the cells to confirm that the cancer is a homologous recombination-deficient cancer.

To a cancer patient diagnosed with homologous recombination-deficient cancer by the diagnostic technique for homologous recombination-deficient cancer by the present invention, a DNA-damaging anticancer drug such as a PARP inhibitor or a platinum formulation may be preferably administered. To a cancer patient diagnosed as not having homologous recombination-deficient cancer, an anticancer drug other than a DNA-damaging anticancer drug may be preferably administered.

Examples of a mode of use of the diagnostic agent for homologous recombination-deficient cancer include, but are not limited to, the following modes.
(1) Cancer cells collected from a cancer patient are cultured, and the nucleic acid construct of the present invention is introduced into the cultured cancer cells, followed by investigating whether or not a signal of protein A is detected (or investigating whether or not a cytocidal effect by protein A is found). If desired, non-cancerous cells (such as peripheral blood cells) collected from the same cancer patient may be used as a control. In cases where protein A is a luciferase, a substrate of the luciferase is added for the detection reaction. In cases where protein A is a fluorescent protein, a fluorescence signal may be detected using a luminometer or the like. When the signal of protein A is detected (especially when the level of the detected signal is almost the same as that of control non-cancerous cells), the cancer of the patient can be judged not to be deficient in homologous recombination. When the signal of protein A is not detected, or when a signal lower than that of control non-cancerous cells is detected, the cancer of the patient can be judged to be deficient in homologous recombination.
   The method of measuring the cell survival rate is well known in the art, and the measurement can be easily carried out using a commercially available kit or the like. When cancer cells into which the nucleic acid construct has been introduced show a decreased survival rate (in particular, when the survival rate is as low as that of control non-cancerous cells), the cancer of the patient can be judged not to be deficient in homologous recombination. When no decrease in the survival rate of cancer cells is found, or when a decrease in the cell survival rate of cancer cells is slower than that of control non-cancerous cells, the cancer of the patient can be judged to be deficient in homologous recombination.
(2) The nucleic acid construct of the present invention is administered to a patient, and whether or not a signal of protein A is detected from a cancer lesion is investigated. When the signal is detected, the cancer of the patient can be judged not to be deficient in homologous recombination. When the signal is not detected, a possibility that the cancer of the patient is deficient in homologous recombination is indicated. In this mode, a protein whose intracellular expression is detectable as a signal is used as protein A, and biotoxicity of the protein employed needs to be sufficiently low. The administration of the nucleic acid construct to the patient is preferably carried out by topical administration into the tumor or into the vicinity of the tumor.
(3) Cancer cells present in blood are isolated from a patient, and then concentrated when necessary, followed by introducing the nucleic acid construct of the present invention into the cells and investigating whether or not a signal of protein A is detected (or whether or not a cytocidal effect due to protein A is found) (in cases of leukemia).
(4) A nucleic acid construct that employs a gene sequence encoding a secretory luciferase as the gene sequence encoding protein A is topically administered into the tumor or into the vicinity of the tumor of the patient. Thereafter, a blood sample is collected, and the presence or absence of luciferase reaction is investigated using the blood sample. In cases where the cancer of the patient is not deficient in homologous recombination, the secretory luciferase is produced from the luciferase gene reconstructed in the cancer cells, and secreted to the outside of the cells. Thus, the luciferase reaction can be detected using the blood sample. In cases where the cancer of the patient is deficient in homologous recombination, the secretory luciferase is not produced, so that the luciferase reaction is not detected in the blood sample.

The nucleic acid construct of the present invention can also be used, for example, as a detection agent for homologous recombination-restored cancer cells. A homologous recombination-restored cancer is a cancer which was once deficient in homologous recombination but has restored its homologous recombination activity as a result of therapeutic treatment or the like. Typical examples of the homologous recombination-restored cancer include cancers that acquired resistance to treatment by a PARP inhibitor (PARP-inhibitor-resistant cancers). The term "homologous recombination-restored cancer" also includes cancers whose *BRCAI* deficiency has been restored (by reverse mutation) to have normalized *BRCA1.* In this mode of use, as the gene sequence encoding protein A, a gene sequence encoding a protein whose intracellular expression is detectable may be preferably used. The specific mode of use is the same as the mode of use of the diagnostic agent for homologous recombination-deficient cancer, but the target cancer patient may be mainly a patient under treatment with a PARP inhibitor or a patient who was once diagnosed with homologous recombination-deficient cancer. The nucleic acid construct of the present invention may be introduced into cancer cells of the patient by the same method as the above-described diagnostic technique for homologous recombination-deficient cancer, and the expression of protein A, preferably the activity of protein A, may be measured. Similarly to the diagnostic method for homologous recombination-deficient cancer, control cells (HR-proficient control cells or HR-deficient control cells, or both) may be used in the detection method for homologous recombination-restored cancer cells. When expression or activity of protein A is detected, it can be judged that homologous recombination-restored cancer cells are detected. In a method of using the detection agent, for example, homologous recombination activity in homologous recombination-deficient cancer cells before restoration of homologous recombination may be measured in advance using a measurement reagent or kit for homologous recombination activity according to the present invention, and then whether or not the homologous recombination is restored may be judged by comparison with this measured value. When the homologous recombination activity is increased compared to the past measured value, in other words, when the protein A expression level or activity level is increased compared to the protein A expression level or activity level in the cancer cells measured in the cancer patient using the nucleic acid construct of the present invention in the past, it is indicated that the homologous recombination activity has been restored. For a patient diagnosed with homologous recombination-restored cancer, for example, administration of a PARP inhibitor may be stopped, and treatment with another anticancer drug may be started instead (in cases where the patient is under treatment with the PARP inhibitor). One option of the other anticancer drug is the laterdescribed therapeutic agent of the present invention for homologous recombination-restored cancer. For a patient diagnosed as not having homologous recombination-restored cancer, for example, treatment with the same anticancer drug may be continued, or treatment with another anticancer drug may be started instead.

The nucleic acid construct of the present invention can also be used, for example, as a companion diagnostic agent for predicting the effect of an anticancer drug on homologous recombination-deficient cancer. Examples of the anticancer drug include DNA-damaging anticancer drugs including PARP inhibitors. Specific examples of modes of the use as a companion diagnostic agent include those described above for the diagnostic agent for homologous recombination-deficient cancer. As the gene sequence encoding protein A, a gene sequence encoding a protein whose intracellular expression is detectable may be preferably used, and a gene sequence encoding a protein that acts to decrease the cell survival rate may also be used. In cases where a patient is diagnosed with homologous recombination-deficient cancer, the patient can be judged to be a patient for whom a desired anticancer effect is likely to be obtained with a DNA-damaging anticancer drug such as a PARP inhibitor. Thus, to such a cancer patient, a DNA-damaging anticancer drug may be preferably administered.

DNA-damaging anticancer drugs include inhibitors of proteins having a synthetic lethal (or synthetic growth delay) relationship with homologous recombination, and agents that induce DNA damage repairable by homologous recombination. Examples of the former include inhibitors of proteins such as PARP, PolQ, and Rad52, and examples of the latter include camptothecin, cisplatin, and PARP inhibitors, although they are not limited to these.

Specific examples of the PARP inhibitors include, but are not limited to, Olaparib, Rucaparib, Niraparib, Veliparib, Talazoparib, PJ34 (Visochek et al., Oncotarget, 2019, Vol. 10, (No. 58), pp: 6269-6282), and NU1025 (CAS 90417-38-2).

A PolQ inhibitor can be obtained by, for example, the method described in JP 2017-201978 A. PolQ inhibitors include a variety of PolQ inhibitors that may be discovered in the future by this method, or by methods different therefrom.

Examples of Rad52 inhibitors include, but are not limited to, 6-HydroxyDL-dopa, AICAR (CAS 2627-69-2), AICAR 50 phosphate (ZMP), 6-HydroxyDL-dopa (CAS 21373-30-8), D-103, D-G23, (-)-Epigallocatechin, EGC (CAS 490-46-0), and NP-004255 (CAS 23094-69-1) (Hengel SR et al. Cell Chem Biol. 2017 Sep 21;24(9): 1101-1119.).

Further examples of the DNA-damaging anticancer drugs include inhibitors related to DDR, which is a target of synthetic lethality (see, for example, Gourley C et al. J Clin Oncol. 2019 Sep 1;37(25):2257-2269., Gourley C et al. J Clin Oncol. 2019 Sep 1;37(25):2257-2269, and Ashworth A and Lord CJ. Nat Rev Clin Oncol. 2018 Sep;15(9):564-576.). Specific examples thereof include, but are not limited to, CBP-501, Prexasertib, GDC-0575, and SRA-737, which target CHK1/2; AZD-1775, which targets WEE1; AZD-6738, M-4344, and M6620 (VX-970), which target ATR; CC-115, LY-3023414, AsiDNA, and M-3814, which target DNA-PK; and AZD-0156, which targets ATM (Gourley C et al., 2019 (described above)).

Further, specific examples of the DNA-damaging anticancer drugs include platinum formulations such as cisplatin, carboplatin, and oxaliplatin (DNA synthesis inhibition); pyrimidine-based drugs such as fluorouracil and gemcitabine (DNA synthesis inhibition); camptothecin-based drugs such as irinotecan and topotecan (DNA synthesis inhibition); epipodophyllotoxin-based drugs such as etoposide (DNA synthesis inhibition); anthracycline-based drugs such as doxorubicin, epirubicin, pirarubicin (DNA synthesis inhibition); and alkylating agents such as cyclophosphamide and ifosfamide (DNA synthesis inhibition). The term "DNA-damaging anticancer drugs" may also include vinca alkaloid-based drugs such as vinblastine, vincristine, vindesine, and vinorelbine (cell division inhibition); and taxane-based drugs such as paclitaxel and docetaxel (apoptosis-inducing agents). However, the DNA-damaging anticancer drugs are not limited to the examples described above.

The nucleic acid construct of the present invention can also be used, for example, as a therapeutic agent for homologous recombination-restored cancer. The definition of the homologous recombination-restored cancer is as described above, and it includes cancers that acquired resistance to treatment with a PARP inhibitor (PARP inhibitor-resistant cancers) and cancers whose *BRCA1* deficiency has been restored (by reverse mutation) to have normalized *BRCA1.* In this mode of use, the gene sequence encoding protein A is a gene sequence encoding a protein that acts to decrease the cell survival rate. By the nucleic acid construct of the present invention, cancer cells having homologous recombination activity can be killed by the action of protein A. Therefore, the construct enables treatment of homologous recombination-restored cancer such as a PARP inhibitor-resistant cancer, for which no treatment method is available at present.

The nucleic acid construct of the present invention may also be used for, for example, determining or predicting whether or not a gene mutation (especially a mutation in an HR-related gene) identified in a cancer patient is a pathogenic mutation that deteriorates homologous recombination activity.

When a mutation is identified in a certain gene X (the gene X is preferably an HR-related gene) in cancer cells of a cancer patient, first, an expression vector that expresses a mutant gene X having the same mutation is constructed. The mutant gene X can be obtained by extracting mRNA from cancer cells collected from the cancer patient, and performing reverse transcription PCR using a primer set targeting the gene X. In addition, an expression vector that expresses the wild-type gene X having no mutation is also provided. The wild-type gene X can be prepared from, for example, a known cell line having normal homologous recombination activity (for details, see the above description on the HR-proficient control cells), or non-cancerous cells of the patient. In cases where an expression vector that expresses the wild-type gene X is already known, the known expression vector may be used without newly constructing an expression vector.

Subsequently, each of the mutant gene X expression vector and the wild-type gene X expression vector is introduced into cells deficient in gene X, and the nucleic acid construct of the present invention is further introduced thereto. The introduction of each expression vector and the introduction of the nucleic acid construct may be carried out in an arbitrary order. Cells deficient in gene X can be prepared by knocking out gene X in cells having normal homologous recombination activity. Since the technique for knockout of a specific gene has been established, those skilled in the art can easily prepare gene X knockout cells using a well-known technique. Or, when there is a known cell line whose function of gene X is known to be completely deficient, such a known cell line may be used.

Subsequently, in cells in which each expression vector and the nucleic acid construct are introduced, expression of protein A is measured. In addition, measurement of expression of protein A may be carried out also in cells prepared by introducing the nucleic acid construct of the present invention into gene X-deficient cells in which an empty vector comprising no insert is introduced, or into gene X-deficient cells in which no vector is introduced. As described above, the measurement of expression of protein A is preferably measurement of the activity of protein A. Based on the expression level of protein A, whether or not the mutation in gene X is a pathogenic mutation (whether or not the mutation causes deficiency of homologous recombination activity) can be judged. This judgment can be carried out according to the same criteria as in the above-described measurement technique for homologous recombination activity. When the expression level or activity level of protein A in the cells in which the mutant gene X is introduced is lower than the expression level or activity level of protein A in the cells in which the wild-type gene X is introduced, the mutation in gene X is indicated to be a pathogenic mutation that deteriorates homologous recombination activity.

In this pathogenic-mutation prediction technique, as protein A, a protein whose intracellular expression is detectable preferably as a signal may be preferably used, but a protein that acts to decrease the cell survival rate may also be used.

In cases where the agent of the present invention is administered to a patient, the administration route may be oral administration or parenteral administration. In general, parenteral administration such as intravenous administration, intraarterial administration, subcutaneous administration, or intramuscular administration is preferred. The administration may be systemic administration, administration into the tumor or into the vicinity of the tumor, or administration to a regional lymph node of the tumor. However, in cases of a therapeutic agent for homologous recombination-restored cancer, the agent is preferably used by topical administration into the tumor or into the vicinity of the tumor since its systemic administration causes production of the cytocidal effect also on normal non-cancerous cells having homologous recombination activity. The dose is not limited, and may be about 1 pg to 10 g, for example, about 0.01 mg to 100 mg, in terms of the amount of the nucleic acid construct per day for the patient. The agent may be administered in a single dose or several divided doses per day. The agent may be administered every day, or may be administered every several days or every several weeks.

The dosage form of the agent of the present invention to be administered to the patient is not limited. Depending on the administration route, the agent may be prepared by appropriately mixing the nucleic acid construct of the present invention with an additive(s) such as a pharmaceutically acceptable carrier, diluent, or excipient. Examples of the formulation include parenteral formulations such as drops, injection solutions, suppositories, and inhalants; and oral formulations such as tablets, capsules, granules, powders, and syrups. Preparation methods and additives that can be used are well known in the field of pharmaceutical formulations, and any of the methods and additives may be used.

In cases of a diagnostic agent, a companion diagnostic agent, or a detection agent to be used in vitro, the amount of the agent used for the treatment of the cells may be, for example, about 10 pg to 1 mg, or for example, about 0.1 ng to 100 µg in terms of the amount of the nucleic acid construct per 1,000,000 cells. The dosage form may be a liquid agent supplemented, when desired, with an additive(s) or the like useful for stability or the like of the nucleic acid construct, or may be a powder agent prepared by freeze-drying of the nucleic acid construct.

In cases where the nucleic acid construct of the present invention is used as a therapeutic agent or a diagnostic agent, examples of the target cancer patient include cancer patients of various mammals such as humans, dogs, cats, ferrets, hamsters, mice, rats, horses, and pigs. The nucleic acid construct of the present invention is applicable to various animal species without changing the components except that the origin of the promoter is changed when necessary.

### EXAMPLES

The present invention is described below more concretely by way of Examples. However, the present invention is not limited to the following Examples.

### A. Method of Preparation of Vectors (DNA Constructs)

### A-1. Construction of pCMV-DR-DTA Construct (Version 1)

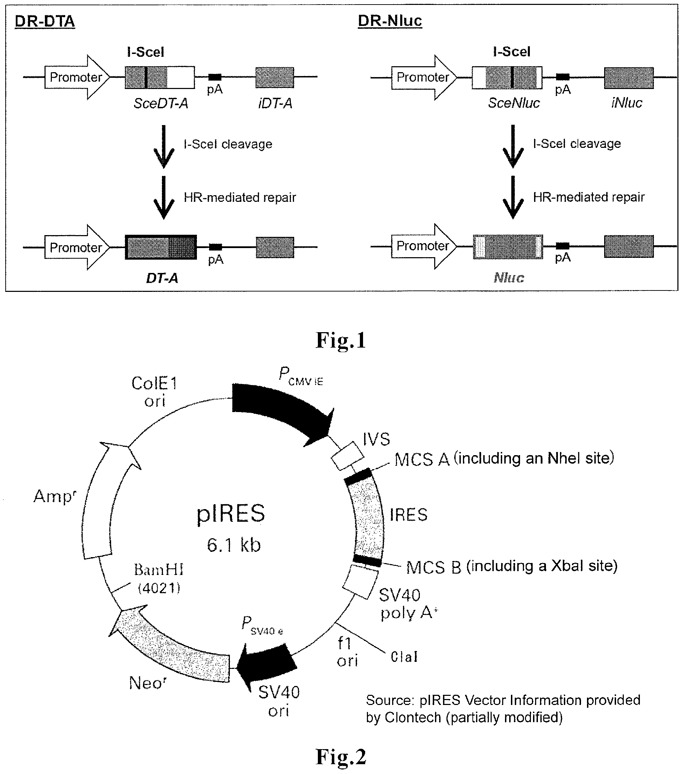

As two fragments of the *DT-A* gene used for pCMV-DR-DTA, a 588-bp SceDTA fragment (SEQ ID NO:15) prepared by replacing the region of positions 133 to 153 in the *DT-A* gene ORF sequence (SEQ ID NO:13) with "stop codon (TGA) + I-SceI recognition sequence", and a 357-bp iDTA fragment (SEQ ID NO:18) consisting of the region of positions 1 to 357 in the *DT-A* gene ORF sequence, containing the above replaced region, were obtained by PCR amplification using pMC1DT-ApA (KURABO, Osaka, Japan) as a template. As a polymerase for the PCR, PrimeSTAR HS (trade name) DNA Polymerase (Takara Bio Inc.) was used. The primers used are shown below in Table 1. In the amplification of the SceDTA fragment, two fragments were separately amplified to obtain a 5'SceDTA fragment and a 3' SceDTA fragment. In the amplification of the 5'SceDTA fragment, DTA-Fw and Sce-5'DTA-Rv were used to amplify a DNA fragment having a structure in which a sequence for In-Fusion reaction was added to the 5'-end of the 5'SceDTA fragment, and in which "stop codon (TGA) + I-SceI recognition sequence" were added to the 3'-end of the 5'SceDTA fragment. In the amplification of the 3'SceDTA fragment, Sce-3'DTA-Fw and DTA-Rv were used to amplify a DNA fragment having a structure in which an I-Scel recognition sequence was added to the 5'-end of the 3' SceDTA fragment, and in which a stop codon (TGA) and a sequence for In-Fusion reaction were added to the 3'-end of the 3'SceDTA fragment. In the amplification of the iDTA fragment, iDTA-Fw and iDTA-Rv were used to amplify a DNA fragment having a structure in which a stop codon (TGA) was linked to the 3'-end of the iDTA fragment, and in which sequences for In-Fusion reaction were added to both ends. Subsequently, pIRES (Takara Bio Inc., Fig. 2) was digested with NheI and XbaI to remove the IRES region, and a fragment containing the CMV promoter and the poly-A addition signal was recovered. Using an In-Fusion HD Cloning Kit (Takara Bio Inc.), the 5'SceDTA fragment and the 3'SceDTA fragment were inserted downstream of the CMV promoter (the site where the IRES region had been present), to obtain pCMV-Sce-DTA, in which [CMV promoter] - [SceDTA fragment] - [poly-A addition signal] were linked together. Finally, pCMV-Sce-DTA was digested with ClaI, and the iDTA fragment was inserted downstream of the poly-A addition signal using an In-Fusion HD Cloning Kit (Takara Bio Inc.), to obtain pCMV-DR-DTA, in which [CMV promoter] - [SceDTA fragment] - [poly-A addition signal] - [iDTA fragment] were linked together. The prepared plasmid was purified using a Qiagen Plasmid Plus Midi Kit (Qiagen K. K.), and linearized by cleavage with I-SceI (New England Biolabs, Ipswich, MA, USA) before using the plasmid in transfection.

### A-2. Construction of pCMV-DR-Nluc Construct (Version 1)

As two fragments of the *Nluc* gene used for pCMV-DR-Nluc, a 513-bp SceNluc fragment (SEQ ID NO:22) prepared by replacing the region of positions 223 to 243 in the *Nluc* gene sequence (SEQ ID NO:20) with "stop codon (TGA) + I-Scel recognition sequence", and a 459-bp iNluc fragment (SEQ ID NO:25) consisting of the region of positions 16 to 474 in the *Nluc* gene sequence, containing the above replaced region, were obtained by PCR amplification using pNL1.1 [Nluc] Vector (Promega, Madison, WI, USA) as a template. As a polymerase for the PCR, PrimeSTAR HS (trade name) DNA Polymerase (Takara Bio Inc.) was used. The primers used are shown below in Table 1. In the amplification of the SceNluc fragment, two fragments were separately amplified to obtain a 5'SceNluc fragment and a 3'SceNluc fragment. In the amplification of the 5'SceNluc fragment, Nluc-Fw (SEQ ID NO:7) and Sce-5'Nluc-Rv (SEQ ID NO:8) were used to amplify a DNA fragment having a structure in which a sequence for In-Fusion reaction was added to the 5'-end of the 5'SceNluc fragment, and in which "stop codon (TGA) + I-SceI recognition sequence" were added to the 3'-end of the 5'SceNluc fragment. In the amplification of the 3'SceNluc fragment, Sce-3'Nluc-Fw (SEQ ID NO:9) and Nluc-Rv (SEQ ID NO:10) were used to amplify a DNA fragment having a structure in which an I-SceI recognition sequence was added to the 5'-end of the 3'SceNluc fragment, and in which a stop codon (TAA) and a sequence for In-Fusion reaction were added to the 3'-end of the 3'SceNluc fragment. In the amplification of the iNluc fragment, iNluc-Fw (SEQ ID NO:11) and iNluc-Rv (SEQ ID NO:12) were used to amplify a DNA fragment having a structure in which a stop codon (TGA) was linked to the 3'-end of the iNluc fragment, and in which sequences for In-Fusion reaction were added to both ends. Subsequently, pIRES (Takara Bio Inc., Fig. 2) was digested with NheI and Xbal to remove the IRES region, and a fragment containing the CMV promoter and the poly-A addition signal was recovered. Using an In-Fusion HD Cloning Kit (Takara Bio Inc.), the 5'SceNluc fragment and the 3'SceNluc fragment were inserted downstream of the CMV promoter (the site where the IRES region had been present), to obtain pCMV-Sce-Nluc, in which [CMV promoter] - [SceNluc fragment] - [poly-A addition signal] were linked together. Finally, pCMV-Sce-Nluc was digested with BamHI, and the iNluc fragment was inserted downstream of the poly-A using an In-Fusion HD Cloning Kit (Takara Bio Inc.), to obtain pCMV-DR-Nluc, in which [CMV promoter] - [SceNluc fragment] - [poly-A addition signal] - [iNluc fragment] were linked together. The prepared plasmid was purified using a Qiagen Plasmid Plus Midi Kit (Qiagen K. K.), and linearized by cleavage with I-SceI (New England Biolabs, Ipswich, MA, USA) before using the plasmid in transfection.

### A-3. Construction of pCMV-DR-Nluc-v2 Construct

As a modified type of the pCMV-DR-Nluc construct (version 1), version 2 in which the iNluc fragment was not located at the most downstream position but was located upstream of the promoter was constructed.

Two fragments of the *Nluc* gene used for pCMV-DR-Nluc-v2 (a 513-bp SceNluc fragment (SEQ ID NO:22) and a 459-bp iNluc fragment (SEQ ID NO:25)) were obtained by amplification from pNL1.1 [Nluc] Vector in the same manner as in A-2. However, for the amplification of the iNluc fragment, iNluc-Fw2 (SEQ ID NO:27) and iNluc-Rv2 (SEQ ID NO:28) were used instead of iNluc-Fw and iNluc-Rv so as to change the sequences added for In-Fusion reaction. pIRES (Takara Bio Inc., Fig. 2) was digested with NheI and XbaI to remove the IRES region, and a fragment containing the CMV promoter and the poly-A addition signal was recovered. Using an In-Fusion HD Cloning Kit (Takara Bio Inc.), the 5'SceNluc fragment and the 3'SceNluc fragment were inserted downstream of the CMV promoter (the site where the IRES region had been present), to obtain pCMV-Sce-Nluc, in which [CMV promoter] - [SceNluc fragment] - [poly-A addition signal] were linked together. Finally, pCMV-Sce-Nluc was digested with BglII, and the iNluc fragment was inserted upstream of the CMV promoter using an In-Fusion HD Cloning Kit (Takara Bio Inc.), to obtain pCMV-DR-Nluc-v2, in which [iNluc fragment] - [CMV promoter] - [SceNluc fragment] - [poly-A addition signal] were linked together. The prepared plasmid was purified using a Qiagen Plasmid Plus Midi Kit (Qiagen K. K., Tokyo, Japan), and linearized by cleavage with I-SceI (New England Biolabs, Ipswich, MA, USA) before using the plasmid in transfection.

### A-4. Construction of pCMV-DR-DTA-v2 Construct

As a modified type of the pCMV-DR-DTA construct (version 1), version 2 in which the iDTA fragment was not located at the most downstream position but was located upstream of the promoter was constructed.

Two fragments of the *DT-A* gene used for pCMV-DR-DTA-v2 (a 588-bp SceDTA fragment (SEQ ID NO:15) and a 357-bp iDTA fragment (SEQ ID NO:18)) were obtained by amplification from pMC1DT-ApA in the same manner as in A-1. However, for the amplification of the iDTA fragment, iDTA-Fw2 (SEQ ID NO:29) and iDTA-Rv2 (SEQ ID NO:30) were used instead of iDTA-Fw and iDTA-Rv so as to change the sequences added for In-Fusion reaction. pIRES (Takara Bio Inc., Fig. 2) was digested with NheI and Xbal to remove the IRES region, and a fragment containing the CMV promoter and the poly-A addition signal was recovered. Using an In-Fusion HD Cloning Kit (Takara Bio Inc.), the 5'SceDTA fragment and the 3' SceDTA fragment were inserted downstream of the CMV promoter (the site where the IRES region had been present), to obtain pCMV-Sce-DTA, in which [CMV promoter] - [SceDTA fragment] - [poly-A addition signal] were linked together. Finally, pCMV-Sce-DTA was digested with BglII, and the iDTA fragment was inserted upstream of the CMV promoter using an In-Fusion HD Cloning Kit (Takara Bio Inc.), to obtain pCMV-DR-DTA-v2, in which [iDTA fragment] - [CMV promoter] - [SceDTA fragment] - [poly-A addition signal] were linked together. The prepared plasmid was purified using a Qiagen Plasmid Plus Midi Kit (Qiagen K. K.), and linearized by cleavage with I-SceI (New England Biolabs) before using the plasmid in transfection.

### A-5. Construction of pCMV-DR-TK Construct

A construct using the *HSV-TK* gene as a suicide gene was constructed.

As two fragments of the *HSV-TK* gene used for pCMV-DR-TK, 1128-bp SceTK fragment (SEQ ID NO:39) prepared by replacing the region of positions 499 to 519 in the *HSV-TK* gene ORF sequence (SEQ ID NO:37) with "stop codon (TGA) + I-Scel recognition sequence", and a 1063-bp iTK fragment (SEQ ID NO:42) consisting of the region of positions 27 to 1089 in the *HSV-TK* gene ORF sequence, containing the above replaced region, were obtained by PCR amplification using an *HSV-TK* gene fragment (GenBank:V00470.1, Kobayashi et al. (2001) Decreased topoisomerase IIalpha expression confers increased resistance to ICRF-193 as well as VP-16 in mouse embryonic stem cells. Cancer Lett. 166(1): 71-77) as a template. As a polymerase for the PCR, PrimeSTAR HS (trade name) DNA Polymerase (Takara Bio Inc.) was used. The primers used are shown below in Table 1. In the amplification of the SceTK fragment, two fragments were separately amplified to obtain a 3'SceTK fragment containing the poly-A addition signal and a 5'SceTK fragment. In the amplification of the 5'SceTK fragment, Sce-5'TK-Fw (SEQ ID NO:31) and Sce-5'TK-Rv (SEQ ID NO:32) were used to amplify a DNA fragment having a structure in which a sequence for In-Fusion reaction was added to the 5'- end of the 5'SceTK fragment, and in which a stop codon (TGA) was added to the 3'- end of the 5'SceTK fragment. In the amplification of the 3'SceTK fragment, Sce-3'TK-Fw (SEQ ID NO:33) and Sce-3'TK-Rv (SEQ ID NO:34) were used to obtain a DNA fragment having a structure in which an I-Scel recognition sequence was added to the 5'-end of the 3'SceTK fragment, and in which a sequence for In-Fusion reaction was added to the 3'-end of the 3'SceTK fragment. In the amplification of the iTK fragment, iTK-Fw (SEQ ID NO:35) and iTK-Rv (SEQ ID NO:36) were used to amplify a DNA fragment having a structure in which sequences for In-Fusion reaction were added to both ends. Subsequently, pIRES (Takara Bio Inc., Fig. 2) was digested with NheI and BamHI, and a fragment containing the CMV promoter was recovered. Using an In-Fusion HD Cloning Kit (Takara Bio Inc.), the 5'SceTK fragment and the 3'SceTK fragment were inserted downstream of the CMV promoter, to obtain pCMV-Sce-TK, in which [CMV promoter] - [SceTK fragment] - [poly-A addition signal] were linked together. Finally, pCMV-Sce-TK was digested with BglII, and the iTK fragment was inserted upstream of the CMV promoter using an In-Fusion HD Cloning Kit (Takara Bio Inc.), to obtain pCMV-DR-TK, in which [iTK fragment] - [CMV promoter] - [SceTK fragment] - [poly-A addition signal] were linked together. The prepared plasmid was purified using a Qiagen Plasmid Plus Midi Kit (Qiagen K. K.), and linearized by cleavage with I-Scel (New England Biolabs) before using the plasmid in transfection.

**[Table 1]**

| Primer name | Sequence (5'→3') | SEQ ID NO |
|---|---|---|
| DTA-Fw | ACTCACTATAGGCTAGCGTCCTCGCCATGGATCCTGATGATGTTG | 1 |
| Sce-5'DTA-Rv | ATTACCCTGTTATCCCTA**TCA**TTGTGTACCAGATTTTGGC | 2 |
| Sce-3'DTA-Fw | TAGGGATAACAGGGTAATAAAGGGTTTTATAGTACCGACAA | 3 |
| DTA-Rv | CCGCCCCGACTCTAGAA**TCA**CAAAGATCGCCTGACACGATTTCCTG | 4 |
| iDTA-Fw | AAATGTGGTAAAATCGATGAATTACAGCTCTTAAGGCTAGAG | 5 |
| iDTA-Rv | ACGCGGATCCTTATCG**TCA**GACTTGCTCCATCAACGGTTCAG | 6 |
| Nluc-Fw | TAATACGACTCACTATAGGCTAG | 7 |
| Sce-5'Nluc-Rv | ATTACCCTGTTATCCCTA**TCA**CTGGCCCATTTGGTCGCCGCTC | 8 |
| Sce-3'Nluc-Fw | TGATAGGGATAACAGGGTAATGTGTACCCTGTGGATGATCATCAC | 9 |
| Nluc-Rv | CCGCCCCGACTCTAGAGTCGCGG | 10 |
| iNluc-Fw | AAATCGATAAGGATCGCTAGCATGAAGATTTCGTTGGGGACTGG | 11 |
| iNluc-Rv | CACCATACGCGGATCCTTA**TCA**GTTGATGGTTACTCGGAACAGC | 12 |
| iNluc-Fw2 | ACATGGCTCGACAGATCTATGAAGATTTCGTTGGGGACTGG | 27 |
| iNluc-Rv2 | GGCCAATATTGAAGATCTTA**TCA**GTTGATGGTTACTCGGAACAGC | 28 |
| iDTA-Fw2 | ACATGGCTCGACAGATCTATGACAGCTCTTAAGGCTAGAGTAC | 29 |
| iDTA-Rv2 | GGCCAATATTGAAGATCTGACTTGCTCCATCAACGGTTC | 30 |
| Sce-5'TK-Fw | CTTAATACGACTCACTATAGGCTAGC | 31 |
| Sce-5'TK-Rv | TTACCCTGTTATCCCTA**TCA**GATGGGATGGCGGTCGAAGATGAG | 32 |
| Sce-3'TK-Fw | GATAGGGATAACAGGGTAATGCCGCGCGGTACCTTATGG | 33 |
| Sce-3'TK-Rv | GAGTGCACCATACGCGGATC | 34 |
| iTK-Fw | ACATGGCTCGACAGATCTATGCGCGTCTGCGTTCGACCAGG | 35 |
| iTK-Rv | GGCCAATATTGAAGATCTGTCGCATATCGTCGGTATGG | 36 |

| | | |
|---|---|---|
| Single-underline: I-Scel recognition sequence Double-underline: A site that anneals to the target gene sequence (iDTA-Fw and iDTA-Fw2 target vector sequences located upstream of the DT-A gene in pMC1DT-ApA, and Nluc-Fw and Nluc-Rv target vector sequences located upstream and downstream of the Nluc gene in pNL1.1 [Nluc] Vector, respectively.) Bold: Sequence for introduction of a stop codon (TGA) | | |

### B. Experiment 1 (Experiment Using Construct of Version 1)

### B-1. Evaluation of Cytocidal Effect in Human Cells, and Method of Luciferase Assay

The human pre-B cell line Nalm-6 and cells derived therefrom were cultured at 37°C in a 5% CO₂ incubator (So et al. (2004) Genetic interactions between BLM and DNA ligase IV in human cells. J. Biol. Chem. 279: 55433-55442).

Acquisition of the *RAD54*/*RAD54B*-double-deficient cells was carried out as described previously (Saito S, Kurosawa A, Adachi N. Mechanistic basis for increased human gene targeting by promoterless vectors-roles of homology arms and Rad54 paralogs. FEBS J. 2017 Sep;284(17):2748-2763. doi: 10.1111/febs. 14137.).

Gene transfection into the Nalm-6 cells and the cells derived therefrom was carried out by the electroporation method according to a past report (Saito S, Maeda R, Adachi N. Dual loss of human POLQ and LIG4 abolishes random integration. Nat Commun. 2017 Jul 11;8:16112. doi: 10.1038/ncommsl6112.). After transfecting 2×10⁶ cells with each construct (1 µg), the cytocidal effect was investigated by one of the following methods.
(1) Growth Inhibition Test
   The cells after the gene transfection were plated on a 24-well dish at 1×10³ cells/ml, and the survival rate of the cells was measured at 24-hour intervals using CellTiter-Glo (CellTiter-Glo (registered trademark) Luminescent Cell Viability Assay, Promega).
(2) Colony Formation Method
   Twenty-four hours after the gene transfection, 200 or 5,000 cells were plated on a 60-mm dish, and colonies were allowed to form for 17 days, followed by counting the number of colonies.

A luciferase assay was carried out as follows. Each construct (1 µg) was transfected into 2×10⁶ cells. The cells after the gene transfer were plated on a 12-well dish at 5×10⁵ cells/ml, and the luciferase activity (RLU value) was measured over time using the Nano-Glo Luciferase Assay System (Promega).

### B-2. Results

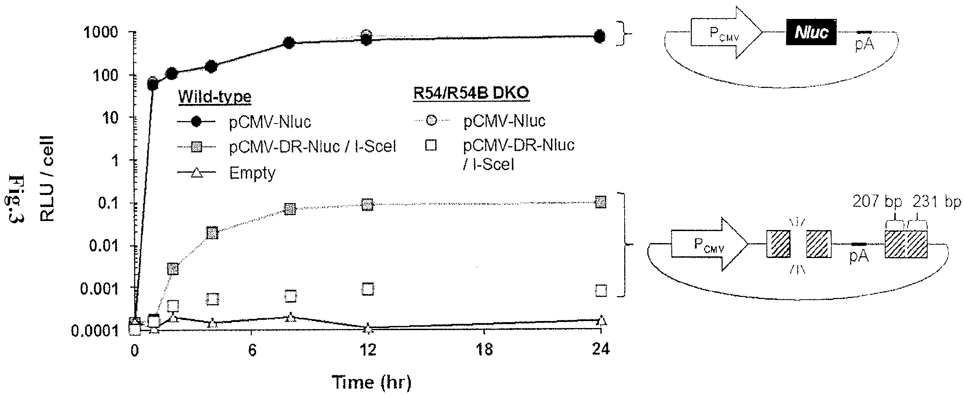

Fig. 3 shows an example of the result of the luciferase assay using the DR-Nluc construct of version 1. In the Nalm-6 cell wild-type line into which pCMV-DR-Nluc was introduced, the luciferase activity increased 2 hours after the gene transfection. In contrast, in the *RAD54*/*RAD54B*-double-deficient line (cells whose homologous recombination ability is lost), the luciferase activity hardly increased by 24 hours after the introduction of pCMV-DR-Nluc. The value of RLU/cell at Hour 24 in the homologous recombination-deficient cells was not more than 1/100 relative to that in the wild-type line. In the figure, pCMV-Nluc shows the result from cells into which a construct that expressed normal *Nluc* gene under the control by the CMV promoter was introduced. These data suggest that the introduction of the pCMV-DR-Nluc construct into the cells allowed production of normal *Nluc* gene by homologous recombination reaction that occurred outside the chromosomes, resulting in transient expression of Nluc protein.

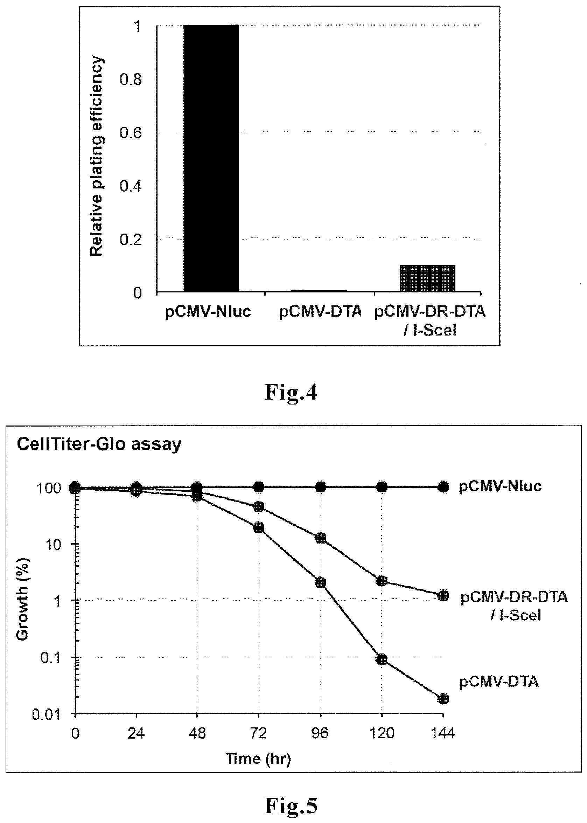

Fig. 4 (the colony formation method) and Fig. 5 (the growth inhibition test) show examples of the results of experiments using the DR-DTA construct of version 1. The survival rate of the cells into which pCMV-DR-DTA was introduced was about 1/100 relative to the survival rate of cells into which pCMV-Nluc was introduced (negative control) (Fig. 5). In Fig. 4 and Fig. 5, pCMV-DTA shows the result from the cells into which a construct that expressed normal *DT-A* gene under the control by the CMV promoter was introduced. Based on the results, the cytocidal effect by pCMV-DR-DTA was confirmed. These results suggest that, by using a DNA construct based on the suicide gene (*DT-A* gene), cells having normal homologous recombination ability can be selectively killed.

### C. Experiment 2

### C-1. Methods

### <Cells and Gene Transfer>

The human pre-B cell line Nalm-6 and cells derived therefrom were cultured at 37°C in a 5% CO₂ incubator (So et al. (2004) Genetic interactions between BLM and DNA ligase IV in human cells. J. Biol. Chem. 279: 55433-55442). Transfection of the Nalm-6 cells was carried out by the electroporation method according to a past report (Saito et al. (2017) Dual loss of human POLQ and LIG4 abolishes random integration. Nat. Commun. 8: 16112).

The human fibrosarcoma cell line HT1080 was cultured at 37°C in a 5% CO₂ incubator (Saito et al. (2015) Construction and applications of exon-trapping gene-targeting vectors with a novel strategy for negative selection. BMC Res. Notes 8, 278). The human breast cancer cell line MDA-MB-436 (ATCC, Manassas, VA, USA) and the human breast cancer cell line HCC1937 (ATCC) were cultured at 37°C in a 5% CO₂ incubator. As a medium, Eagle MEM (Nissui Pharmaceutical Co., Ltd., Tokyo, Japan) supplemented with calf serum (Global Life Science Technologies Japan, Tokyo, Japan; 50 ml of calf serum was added per 500 ml of Eagle MEM), L(+)-glutamine (Fujifilm Wako Pure Chemical Corporation, Osaka, Japan; which was added so that the final concentration should be 2 mM), an MEM non-essential amino acid solution (Fujifilm Wako Pure Chemical Corporation; 10 ml of the solution was added per 500 ml of Eagle MEM), 100 mmol/L sodium pyruvate solution (Fujifilm Wako Pure Chemical Corporation; which was added so that the final concentration should be 1 mM), vitamin B12 (Sigma-Aldrich, St. Louis, MO, USA; which was added so that the final concentration should be 0.15 µM), and 2-mercaptoethanol (Fujifilm Wako Pure Chemical Corporation; which was added so that the final concentration should be 50 µM) was used. Transfection of HT1080 cells, MDA-MB-436 cells, and HCC1936 cells was carried out using jetPEI (Polyplus-transfection, Illkirch, France) according to a protocol provided by the manufacturer.

### <Evaluation of Cytocidal Effect>

After transfecting 2×10⁶ Nalm-6 cells with each construct (1 µg), the cytocidal effect was investigated by either the growth inhibition test method or the colony formation method.
(1) Growth Inhibition Test
   The cells after the gene transfection were plated on a 24-well dish at 1×10⁵ cells/ml, and, 24 hours later, ganciclovir (Fujifilm Wako Pure Chemical Corporation) was added thereto to a final concentration of 500 nM. The survival rate of the cells was measured at 48-hour intervals using CellTiter-Glo (CellTiter-Glo (registered trademark) Luminescent Cell Viability Assay, Promega).
(2) Colony Formation Method
   Twenty-four hours after the gene transfer, 200 or 5,000 cells were plated on a 60-mm dish, and colonies were allowed to form. When pCMV-DR-TK was introduced, 200 or 5,000 cells after the gene transfer were plated on an agar medium supplemented with 500 nM ganciclovir. After culturing the cells for 17 days, grown colonies were counted, and the survival rate was calculated.

### <Luciferase Assay>

A luciferase assay of Nalm-6 cells and their derived cell lines was carried out as follows. Each construct (1 µg) was transfected into 2×10⁶ Nalm-6 cells. The cells after the gene transfer were plated on a 24-well dish at 5×10⁵ cells/ml, and the luciferase activity (RLU value) was measured 24 hours later using the Nano-Glo Luciferase Assay System (Promega).

A luciferase assay of HT1080 cells, MDA-MB-436 cells, and HCC1937 cells was carried out as follows. On a 24-well dish, 5×10⁴ cells were plated and cultured overnight, followed by transfecting the cells with each construct (1 µg). Twenty-four hours after the gene transfection, cells were counted, and the luciferase activity (RLU value) was measured using the Nano-Glo Luciferase Assay System (Promega).

### C-2. Results

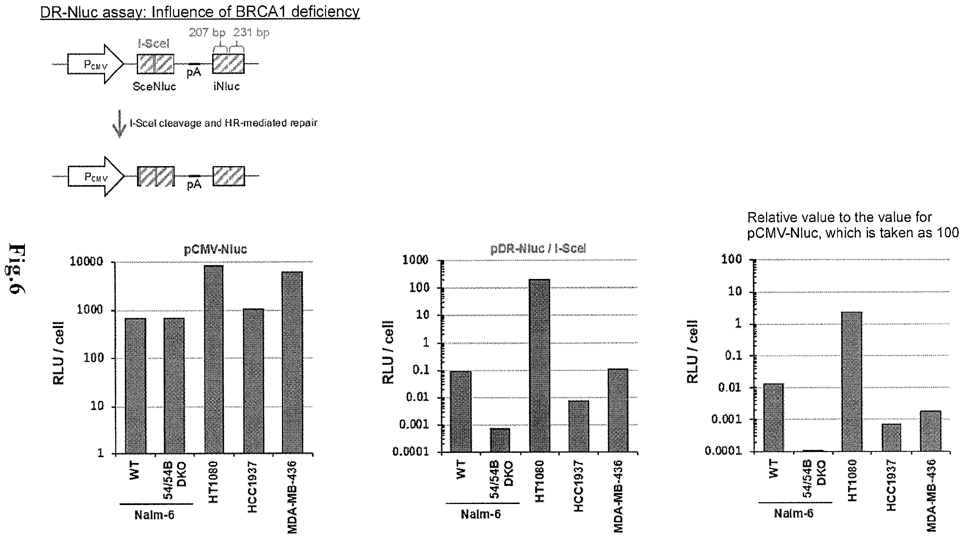

Fig. 6 shows an example of the result of the luciferase assay performed in HT1080 cells, MDA-MB-436 cells, and HCC1937 cells into which the DR-Nluc construct of version 1 was introduced. The RLU values in the *BRCA1*-deficient cells (HCC1937 and MDA-MB-436, which are deficient in homologous recombination) into which pCMV-DR-Nluc was transiently introduced were not more than 1/1000 relative to that in the *BRCA1* normal cells (HT1080, which has normal homologous recombination activity) into which pCMV-DR-Nluc was introduced.

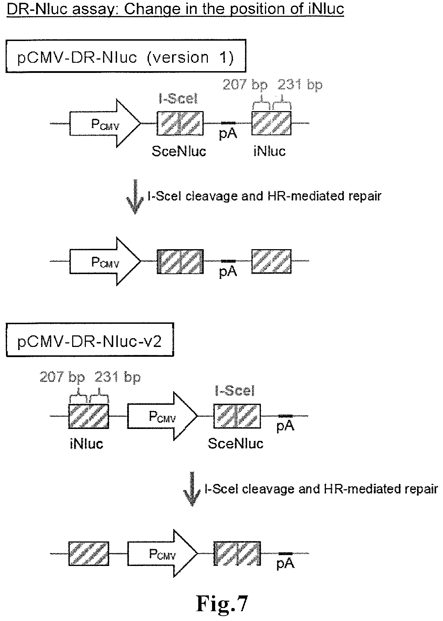

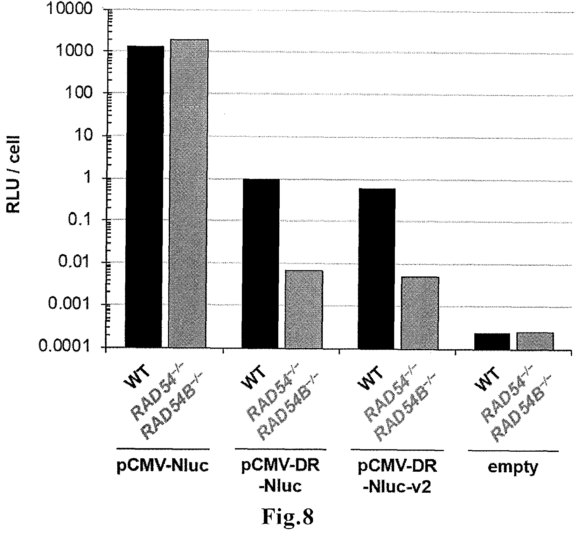

Fig. 7 shows a diagram illustrating the difference between version 1 and 2 DR-Nluc constructs, and Fig. 8 shows an example of the result of the luciferase assay using the DR-Nluc-v2 construct. It was confirmed that the change in the position of iNluc does not cause a change in the RLU value of the luciferase whose expression is dependent on HR, and hence that the position of iNluc does not affect the HR frequency.

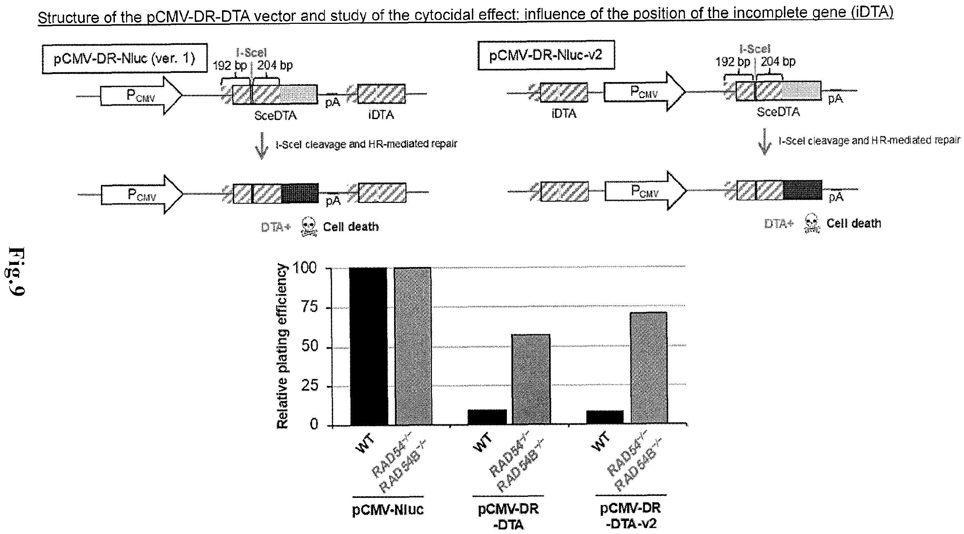

Fig. 9 shows an example of the result of investigation of the cytocidal effect of the DR-DTA-v2 construct by the colony formation method, together with the result of an experiment using the DR-DTA construct of version 1 (the result from the cells to which pCMV-DTA was introduced as a positive control is omitted). It was confirmed that the change in the position of iDTA does not affect the cytocidal effect on cells having normal homologous recombination ability, and that such a change still allows production of a cytocidal effect which is similar to that of version 1.

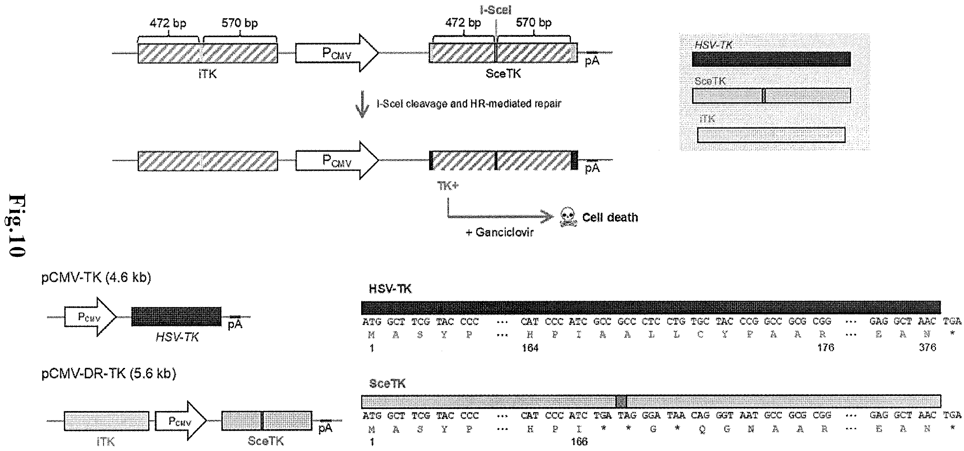

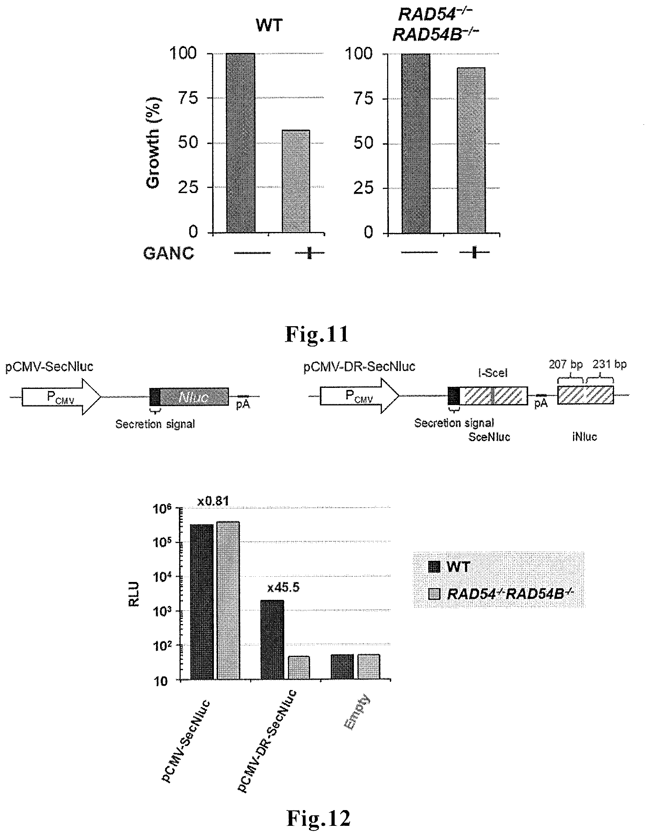

Fig. 10 shows a diagram illustrating the DR-TK construct. Fig. 11 shows an example of the result of investigation of the cytocidal effect of the DR-TK construct by the growth inhibition test. The survival rate of the cells 6 days after the gene transfection is expressed as a relative value with respect to the rate observed without addition of ganciclovir, which is taken as 100. It was confirmed that use of *HSV-TK* as a suicide gene is effective similarly to the case where *DT-A* gene is used.

### D. Experiment 3

### D-1. Construction of pCMV-DR-SecNluc Construct

For preparation of a vector that expresses an *Nluc* gene to which a secretory signal is added, a secretory signal sequence derived from human interleukin 6 (IL6) was prepared by artificial gene synthesis (pIL6-Nluc). The pIL6-Nluc prepared by the artificial gene synthesis was digested with SmaI and EcoNI, and a fragment containing the secretory signal sequence was recovered. The recovered fragment containing the secretory signal sequence was mixed with a DNA fragment obtained by digesting pCMV-Nluc with Smal and EcoNI, and the fragment containing the secretory signal sequence was inserted upstream of the *Nluc* gene using a DNA Ligation Kit <Mighty Mix> (Takara Bio Inc.), to obtain pCMV-SecNluc, in which [CMV promoter] - [secretory signal] - [Nluc gene] - [poly-A addition signal] were linked together. The base sequence of the [secretory signal] - [Nluc gene] portion (SecNluc gene) is shown in SEQ ID NO:44 (wherein positions 1 to 87 correspond to the DNA sequence encoding the secretory signal), and the amino acid sequence encoded thereby is shown in SEQ ID NO:45 (wherein the amino acids at positions 1 to 29 correspond to the secretory signal sequence).

In order to add the secretory signal sequence to the [SceNluc fragment] portion (SceNluc gene) of the pCMV-Sce-Nluc prepared in A-2, pCMV-SecNluc was digested with Xhol and EcoNI, and a fragment containing the secretory signal sequence was recovered. Subsequently, pCMV-DR-Nluc was digested with XhoI and EcoNI, and a fragment containing the CMV promoter was recovered. The recovered fragment containing the secretory signal was mixed with the fragment containing the CMV promoter, and the fragment containing the secretory signal sequence was inserted upstream of the SceNluc gene using a DNA Ligation Kit <Mighty Mix> (Takara Bio Inc.), to obtain pCMV-Sce-SecNluc, in which [CMV promoter] - [secretory signal] - [SceNluc fragment] - [poly-A addition signal] were linked together. Finally, pCMV-Sce-SecNluc was digested with BamHI, and the iNluc fragment prepared in A-2 was inserted downstream of the poly-A addition signal using an In-Fusion HD Cloning Kit (Takara Bio Inc.), to obtain pCMV-DR-SecNluc, in which [CMV promoter] - [secretory signal] - [SceNluc fragment] - [poly-A addition signal] - [iNluc fragment] were linked together (Fig. 12). The base sequence of the [secretory signal] - [SceNluc fragment] portion (SceSecNluc gene) is shown in SEQ ID NO:46 (wherein positions 1 to 87 correspond to the DNA sequence encoding the secretory signal), and the amino acid sequence encoded thereby is shown in SEQ ID NO:47 (wherein the amino acids at positions 1 to 29 correspond to the secretory signal sequence) and SEQ ID NO:48. The prepared plasmid was purified using a Qiagen Plasmid Plus Midi Kit (Qiagen K. K.), and linearized by cleavage with I-Scel (New England Biolabs, Ipswich, MA, USA) before using the plasmid in transfection.

### D-2. Cells and Gene Transfer, and Luciferase Assay

### D-2-1. Cells and Gene Transfer

The human colorectal cancer-derived cell line HCT116 (Horizon Discovery, Cambridge, UK) and MLH1+ HCT116 cells (Horizon Discovery) were cultured at 37°C in a 5% CO₂ incubator. As a medium, Dulbecco's modified Eagle medium (Nissui Pharmaceutical Co., Ltd., Tokyo, Japan) supplemented with calf serum (Global Life Science Technologies Japan, Tokyo, Japan; 50 ml of calf serum was added per 500 ml of Eagle MEM) and L(+)-glutamine (Fujifilm Wako Pure Chemical Corporation, Osaka, Japan; which was added so that the final concentration should be 2 mM) was used. Transfection was carried out using jetPEI (Polyplus-transfection, Illkirch, France) according to a protocol provided by the manufacturer.

### D-2-2. Luciferase Assay

A luciferase assay ofHCT116 cells and MLH1+ HCT116 cells was carried out as follows. On a 24-well dish, 5×10⁴ cells were plated and cultured overnight, followed by transfecting the cells with each construct (1 µg). Twenty-four hours after the gene transfection, the supernatant (50 µl) of the cell culture was subjected to measurement of luciferase activity (RLU value) using the Nano-Glo Luciferase Assay System (Promega).

### D-3. Results

The result of the measurement of the luciferase activity using the culture supernatant is shown in Fig. 13. The result obtained was similar to that obtained with a non-secretory luciferase construct. Use of a secretory luciferase eliminates the necessity of the cell lysis treatment, so that the assay can be simply carried out using a culture supernatant.

## Claims

1. A nucleic acid construct comprising the following (1) to (3):
(1) a promoter region;
(2) a mutant gene sequence containing a cleavage site in a gene sequence encoding a protein; and
(3) a base sequence composed of a first homologous region and a second homologous region and capable of replacing, by homologous recombination, a partial region in the mutant gene sequence of (2), the partial region containing the cleavage site, the base sequence being any one of the following (i) to (iii):
(i) a continuous partial sequence in the gene sequence encoding the protein, the continuous partial sequence containing upstream and downstream regions adjacent to the cleavage site in the mutant gene sequence of (2);
(ii) a base sequence which is homologous to the partial sequence of (i) and encodes the same amino acid sequence as that encoded by said partial sequence; and
(iii) a continuous partial sequence in a gene sequence encoding a protein whose sequence is 80% or more identical to that of the previously-mentioned protein and which has the same activity as the previously-mentioned protein, the continuous partial sequence being a base sequence homologous to the partial sequence of (i).

2. The nucleic acid construct according to claim 1, wherein the (2) contains a stop codon upstream of the cleavage site.

3. The nucleic acid construct according to claim 1, wherein the sequence of (3) is a continuous partial sequence in the gene sequence encoding the protein, the continuous partial sequence containing upstream and downstream regions adjacent to the cleavage site in the mutant gene sequence of (2).

4. The nucleic acid construct according to claim 2, wherein the sequence of (3) is a continuous partial sequence in the gene sequence encoding the protein, the continuous partial sequence containing the stop codon, and upstream and downstream regions adjacent to the cleavage site, in the mutant gene sequence of (2).

5. The nucleic acid construct according to any one of claims 1 to 4, wherein the sequences of (ii) and (iii) is 90% or more homologous to the partial sequence of (i).

6. The nucleic acid construct according to any one of claims 1 to 5, wherein each of the first homologous region and the second homologous region in the sequence of (3) has a strand length of at least 20 bases.

7. The nucleic acid construct according to any one of claims 1 to 6, comprising a poly-A addition signal between the mutant gene sequence of (2) and the sequence of (3).

8. The nucleic acid construct according to any one of claims 1 to 7, wherein the cleavage site is a restriction enzyme recognition site.

9. The nucleic acid construct according to any one of claims 1 to 8, wherein the nucleic acid construct is a circular nucleic acid construct comprising (2) and (3) in this order downstream of (1), or a linear nucleic acid construct prepared by cleaving the circular nucleic acid construct at the cleavage site; or a linear nucleic acid construct comprising (2) and (3) in this order downstream of (1).

10. The nucleic acid construct according to any one of claims 1 to 8, wherein the nucleic acid construct is a circular nucleic acid construct comprising (2) downstream of (1), and comprising (3) upstream of (1), or a linear nucleic acid construct prepared by cleaving the circular nucleic acid construct at the cleavage site; or a linear nucleic acid construct comprising (2) downstream of (1), and comprising (3) upstream of (1).

11. The nucleic acid construct according to any one of claims 1 to 10, wherein the gene sequence is a gene sequence encoding a protein that acts to decrease cell survival rate or a protein whose intracellular expression is detectable.

12. The nucleic acid construct according to claim 11, wherein the gene sequence is a sequence of a suicide gene, a DNA damage-inducing gene, a DNA repair-inhibiting gene, a luminescent enzyme gene, a fluorescent protein gene, a cell surface antigen gene, a secretory protein gene, or a membrane protein gene.

13. A linear nucleic acid construct comprising the following (a) to (d) in the order of (c), (d), (a), and (b) from the upstream side toward the downstream side:
(a) a promoter region;
(b) an upstream-side region of a gene sequence encoding a protein;
(c) a downstream-side region of the gene sequence; and
(d) a base sequence composed of a first homologous region and a second homologous region and capable of replacing the upstream-side region and the downstream-side region by homologous recombination, the base sequence being any one of the following (d-1) to (d-3):
(d-1) a continuous partial sequence in the gene sequence encoding the protein, the continuous partial sequence containing a region that spans the upstream-side region and the downstream-side region;
(d-2) a base sequence which is homologous to the partial sequence of (d-2) and encodes the same amino acid sequence as that encoded by said partial sequence; and
(d-3) a continuous partial sequence in a gene sequence encoding a protein whose sequence is 80% or more identical to that of the previously-mentioned protein and which has the same activity as the previously-mentioned protein, the continuous partial sequence being a base sequence homologous to the partial sequence of (d-1).

14. A measurement reagent for homologous recombination activity, the measurement reagent comprising the nucleic acid construct according to any one of claims 1 to 13, wherein the gene sequence is a gene sequence encoding a protein whose intracellular expression is detectable.

15. A measurement kit for homologous recombination activity, the kit comprising the measurement reagent according to claim 14.

16. A screening system for an agent that affects homologous recombination activity, the screening system comprising the nucleic acid construct according to any one of claims 1 to 13.

17. A diagnostic agent for homologous recombination-deficient cancer, the agent comprising the nucleic acid construct according to any one of claims 1 to 13.

18. A detection agent for homologous recombination-restored cancer cells, the agent comprising the nucleic acid construct according to any one of claims 1 to 13, wherein the gene sequence is a gene sequence encoding a protein whose intracellular expression is detectable.

19. The detection agent according to claim 18, wherein the homologous recombination-restored cancer cells are PARP inhibitor-resistant cancer cells.

20. A companion diagnostic agent for predicting an effect of an anticancer drug on homologous recombination-deficient cancer, the agent comprising the nucleic acid construct according to any one of claims 1 to 13.

21. The companion diagnostic agent according to claim 20, wherein the anticancer drug is a DNA-damaging anticancer drug.

22. The companion diagnostic agent according to claim 21, wherein the DNA-damaging anticancer drug is a PARP inhibitor.

23. A therapeutic agent for homologous recombination-restored cancer, the agent comprising the nucleic acid construct according to any one of claims 1 to 13, wherein the gene sequence is a gene sequence encoding a protein that acts to decrease cell survival rate.

24. The therapeutic agent according to claim 23, wherein the homologous recombination-restored cancer is PARP inhibitor-resistant cancer.

25. A method of measuring homologous recombination activity in test cells, the method comprising:
introducing a nucleic acid construct according to any one of claims 1 to 13, wherein the gene sequence is a gene sequence encoding a protein whose intracellular expression is detectable, into test cells whose homologous recombination activity is to be measured, and into HR-proficient control cells having normal homologous recombination activity;
measuring the expression levels of the protein in the test cells and the HR-proficient control cells; and
comparing the expression level in the test cells with the expression level in the HR-proficient control cells;
wherein a protein expression in the test cells that is lower than the protein expression in the HR-proficient control cells indicates that the test cells have a lower homologous recombination activity.

26. A method of identifying a candidate of an agent that affects homologous recombination activity, the method comprising:
introducing the nucleic acid construct according to any one of claims 1 to 13 into cells having normal homologous recombination activity, and then treating the cells with each compound in a compound group; or treating cells having normal homologous recombination activity with each compound in a compound group, and then introducing the nucleic acid construct according to any one of claims 1 to 13 into the cells; and
measuring expression of the protein.

27. The method according to claim 26, wherein the protein is a protein whose intracellular expression is detectable as a signal, the method comprising selecting a compound as a candidate of an inhibitor that inhibits homologous recombination activity when the signal of the protein detected is lower in the cells treated with the compound than in the cells not treated with the compound, or selecting a compound as a candidate of an enhancer that promotes homologous recombination activity when the signal of the protein rises more rapidly or a higher signal is detected in the cells treated with the compound compared to the cells not treated with the compound.

28. The method according to claim 26, wherein the protein is a protein that acts to decrease cell survival rate, the method comprising selecting a compound as a candidate of an inhibitor that inhibits homologous recombination activity when the decrease in the cell survival rate is suppressed in the cells treated with the compound compared to the cells not treated with the compound, or selecting a compound as a candidate of an enhancer that promotes homologous recombination activity when the decrease in the cell survival rate occurs earlier in the cells treated with the compound than in the cells not treated with the compound.

29. A diagnostic method for homologous recombination-deficient cancer, the method comprising:
introducing the nucleic acid construct according to any one of claims 1 to 13 into cancer cells of a cancer patient; and
measuring expression of the protein.

30. The method according to claim 29, wherein the cancer cells are cells separated from the cancer patient, and the introduction of the nucleic acid construct into the cancer cells is carried out in vitro.

31. The method according to claim 29,
wherein:
the protein is a protein whose intracellular expression is detectable as a signal;
the introduction of the nucleic acid construct into the cancer cells is carried out by administering the nucleic acid construct to the cancer patient; and
whether or not the signal of the protein is detected from a cancer lesion is investigated.

32. The method according to claim 29,
wherein:
the protein is a secretory protein whose intracellular expression is detectable;
the introduction of the nucleic acid construct into the cancer cells is carried out by administering the nucleic acid construct to the cancer patient; and
the activity of the protein in blood separated from the patient after the administration of the nucleic acid construct is measured.

33. A detection method for homologous recombination-restored cancer cells, the method comprising:
introducing the nucleic acid construct according to any one of claims 1 to 13 into cancer cells of a cancer patient that is a patient under treatment with a PARP inhibitor or that is a patient who was once diagnosed with homologous recombination-deficient cancer; and
measuring expression of the protein.

34. The method according to claim 33, wherein the cancer cells are cells separated from the patient, and the introduction of the nucleic acid construct into the cancer cells is carried out in vitro.

35. The method according to claim 33,
wherein:
the protein is a protein whose intracellular expression is detectable as a signal;
the introduction of the nucleic acid construct into the cancer cells is carried out by administering the nucleic acid construct to the patient; and
whether or not the signal of the protein is detected from a cancer lesion is investigated.

36. A method of predicting an effect of an anticancer drug on homologous recombination-deficient cancer, the method comprising:
introducing the nucleic acid construct according to any one of claims 1 to 13 into cancer cells of a cancer patient; and
measuring expression of the protein.

37. The method according to claim 36, wherein the anticancer drug is a DNA-damaging anticancer drug.

38. The method according to claim 37, wherein the DNA-damaging anticancer drug is a PARP inhibitor.

39. The method according to any one of claims 36 to 38, wherein the cancer cells are cells separated from the patient, and the introduction of the nucleic acid construct into the cancer cells is carried out in vitro.

40. The method according to any one of claims 36 to 38,
wherein:
the protein is a protein whose intracellular expression is detectable as a signal;
the introduction of the nucleic acid construct into the cancer cells is carried out by administering the nucleic acid construct to the patient; and
whether or not the signal of the protein is detected from a cancer lesion is investigated.

41. The method according to any one of claims 36 to 38,
wherein:
the protein is a secretory protein whose intracellular expression is detectable;
the introduction of the nucleic acid construct into the cancer cells is carried out by administering the nucleic acid construct to the cancer patient; and
activity of the protein in blood separated from the patient after the administration of the nucleic acid construct is measured.

42. A therapeutic method for homologous recombination-restored cancer, the method comprising administering a nucleic acid construct according to any one of claims 1 to 13, wherein the gene sequence is a gene sequence encoding a protein that acts to decrease cell survival rate, to a patient having the homologous recombination-restored cancer.

43. The method according to claim 42, wherein the nucleic acid construct is topically administered into a tumor or into the vicinity of a tumor of the patient.

44. The method according to claim 42 or 43, wherein the homologous recombination-restored cancer is PARP inhibitor-resistant cancer.

45. A method of predicting whether or not a gene mutation identified in a cancer patient is a pathogenic mutation that deteriorates homologous recombination activity, the method comprising:
constructing an expression vector that expresses a mutant gene having the same mutation as the previously-mentioned mutation;
providing an expression vector that expresses a wild-type gene not having the mutation;
introducing each of the mutant-gene expression vector and the wild-type-gene expression vector, and also introducing the nucleic acid construct according to any one of claims 1 to 11, into cells deficient in the gene; and
measuring expression of the protein in the cells in which each vector and the nucleic acid construct are introduced;
wherein the mutation is indicated to be a pathogenic mutation that deteriorates homologous recombination activity when the expression level of the protein in the cells in which the mutant-gene expression vector is introduced is lower than the expression level of the protein in the cells in which the wild-type-gene expression vector is introduced.
